# EUROPEAN PATENT APPLICATION

(11) **EP 4 198 038 A1**
(43) Date of publication of application: **21.06.2023**
(21) Application number: 21214560.1
(22) Date of filing: 14.12.2021
(51) Int. Cl.: C07F 7/18, C07F 7/08, C07D 205/04, C07D 205/12, C07D 211/96, C07D 307/20, C07D 307/94, C07D 309/10, C07D 319/06, C07D 491/107, C07D 493/10, C07D 493/14

(54) **PROCESS FOR C-H INSERTION BY GEM-HYDROGENATION OF AN INTERNAL ALKYNE**

(71) Applicant: Studiengesellschaft Kohle mbH, 45470 Mülheim (DE)
(72) Inventor: Fürstner, Alois, 45478 Mülheim (DE); Peil, Sebastian, 45470 Muelheim (DE)

(57) **Abstract**

The present invention is directed to a process for C-H insertion by the gem-hydrogenation of an internal alkyne.

## Description

The present invention is directed to a process for C-H insertion by gem-hydrogenation of an internal alkyne.

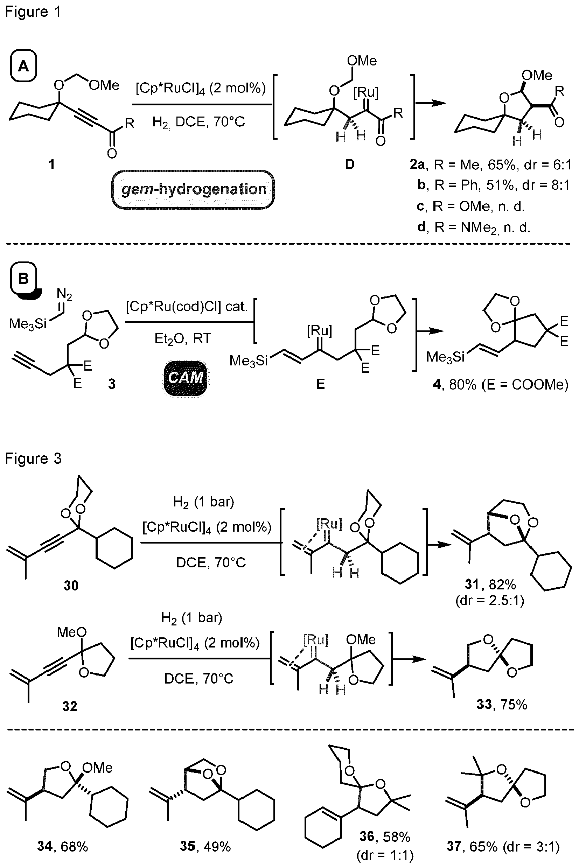

The ability to transfer both H-atoms of H₂ to the same C-atom of an internal alkyne is a fundamentally new reactivity mode. It leads to the formation of a metal carbene flanked by a methylene group, which can react in different ways. It was during a recent application of hydrogenative metathesis to the total synthesis of the marine natural product sinularone F that C-H insertion was observed as yet another possibility for the transient carbene to evolve (Figure 1A) (Peil, S. (2021). Anwendungen der geminalen Hydrierung von Alkinen; PhD Thesis, Technische Universität, Dortmund). However, this then undesired side reaction infringed only in cases such as **1**, in which the derived carbene complex **D** carried a neighboring ketone group; even a flanking ester or an amide did not suffice to upregulate the electrophilicity of the putative intermediate to the necessary extent; these compounds simply got reduced to alkene and alkane. On the other hand, certain pianostool ruthenium carbenes generated by an entirely different route, namely carbene/alkyne metathesis (CAM) (Fuerstner et al, J.Am. Chem. Soc. 2020, 142, 18541-18553), are capable of inserting into *secondary* or *tertiary* C-H bonds of suitably disposed acetals or ethers in moderate to good yields (Figure 1B); insertions into primary C-H bonds have not been described. If one were able to generate the presumed vinylcarbenes of type **E** by *gem*-hydrogenation, one could avoid the use of hazardous diazo-alkanes altogether.

These preliminary data spoke for a narrow window of opportunity, and the goal of establishing a reasonably general hydrogenative C-H insertion protocol seemed (over)ambitious. Moreover, 1,3-enynes are known to bind very tightly to [Cp*Ru]-fragments and had therefore proven problematic substrates in the past in various other reactions effected by such catalysts. It was therefore not clear whether they can be suitably used in *gem*-hydrogenation at all. Not only proved this to be the case, but the ensuing C-H insertion reactions turned out to be truly enabling; most notably, these reactions open access to (spirocyclic) building blocks of immediate relevance in medicinal chemistry. In parallel, the gathered mechanistic information brings the understanding for this type of transformation to a new level.

Based on their considerations, the inventors surprisingly succeeded in providing a process for the gem-hydrogenation of an internal alkyne. The inventors have found that in case of 1,3-enynes bearing a propargylic steering substituent as the substrate, the gem-hydrogenation reaction occurs regioselectively to give a vinyl carbene complex as a reactive intermediate, which is capable of inserting into primary, secondary, or tertiary C-H bonds on the steering group itself or other suitably placed ether, acetal, orthoester, amide, sulfonamide, or carbamate substituents.

In more detail, the invention is directed to a process for C-H insertion by gem-hydrogenation of an internal alkyne wherein a compound of Formula (I) is hydrogenated in the presence of a Ru-catalyst in an organic solvent, whereby a compound of Formula (II) is obtained: 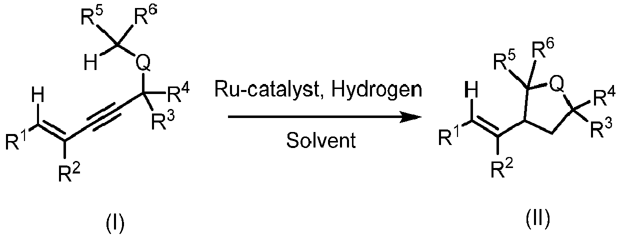 wherein in Formulae (I) and (II):
- R¹ and R² each independently represent
   ∘ hydrogen, optionally with the proviso that at least one of R¹ and R² is not hydrogen;
   ∘ Si(C₁ to C₁₂ alkyl)₃, C(=O)-O-(C₁ to C₁₂ alkyl), or
   ∘ (C₁ to C₁₂)alkyl or hetero(C₁ to C₁₂)alkyl, wherein R¹ and R² may form a ring system with each other via the alkyl or the heteroalkyl,
- R³ and R⁴ each independently represent
   ∘ OSi(C₁ to C₁₂ alkyl)₃, C(=O)-O-(C₁ to C₁₂ alkyl); or
   ∘ (C₁ to C₁₂)alkyl or hetero(C₁ to C₁₂)alkyl, wherein R³ and R⁴ may form a ring system with each other via the alkyl or the heteroalkyl;
- R⁵ and R⁶ each independently represent
   ∘ hydrogen, deuterium,
   ∘ O(C₁ to C₁₂ alkyl), C(=O)-(C₁ to C₁₂ alkyl), C(=O)-O-(C₁ to C₁₂ alkyl),
   ∘ NR^{N1}R^{N2}, wherein R^{N1} represents a protective group selected from an arylsulfonyl group, a (C₁ to C₁₂)alkylsulfonyl group, a (C₁ to C₁₂)alkoxy carbonyl group or -C(=O)-(C₁ to C₁₂ alkyl), and R^{N2} represents a C₁ to C₁₂ alkyl group, or wherein R^{N1} and R^{N2} each represent a C₁ to C₁₂ alkyl group, or
   ∘ (C₁ to C₁₂)alkyl or hetero(C₁ to C₁₂)alkyl, wherein R⁵ and R⁶ may form a ring system with each other via the alkyl or the heteroalkyl,
- wherein R² and R³ may form a ring system with each other if R₁ and R₂ do not form a ring with each other, and/or R⁴ and R⁵ may form a ring system with each other if R₃ and R₄ do not form a ring with each other;
- wherein Q represents CH₂, CH(C₁ to C₁₂ alkyl), C(C₁ to C₁₂ alkyl)₂, O or NR^{N} wherein R^{N} represents a protective group selected from an arylsulfonyl group, a (C₁ to C₁₂)alkylsulfonyl group, a (C₁ to C₁₂)alkoxy carbonyl group or -C(=O)-(C₁ to C₁₂ alkyl),
- with the proviso that, if Q represents CH₂, CH(C₁ to C₁₂ alkyl), or C(C₁ to C₁₂ alkyl)₂, R⁴ and R⁵ each independently represent (C₁ to C₁₂)alkyl or hetero(C₁ to C₁₂)alkyl and form a ring system with each other with O or NR^{N} in the ring wherein R^{N} represents a protective group selected from an arylsulfonyl group, a (C₁ to C₁₂)alkylsulfonyl group, a (C₁ to C₁₂)alkoxy carbonyl group or -C(=O)-(C₁ to C₁₂ alkyl),
   R³ represents (C₁ to C₁₂)alkyl, hetero(C₁ to C₁₂)alkyl or OSi(C₁ to C₁₂ alkyl)₃,
   R⁶ represents hydrogen, deuterium, (C₁ to C₁₂)alkyl or hetero(C₁ to C₁₂)alkyl, and
   R¹ and R² each independently represent:
      ∘ hydrogen, optionally with the proviso that at least one of R¹ and R² is not hydrogen;
      ∘ Si(C₁ to C₁₂ alkyl)₃, C(=O)-O-(C₁ to C₁₂ alkyl), or
      ∘ (C₁ to C₁₂)alkyl or hetero(C₁ to C₁₂)alkyl,
wherein the Ru catalyst is represented by the Formula (III) 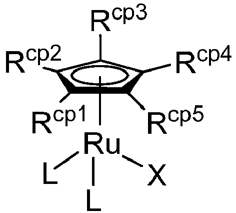 wherein R^{cp1} to R^{cp5} each independently represents C₁-C₅ alkyl which may be branched or linear, C₃-C₅ cycloalkyl, OR^{H} or NR^{H}₂, C(=O)-O-(C₁ to C₁₂ alkyl), C(=O)N(C₁ to C₁₂ alkyl)₂, X represents Cl, Br, I, OTf, BF₄, PF₆, O(C, to C₁₂ alkyl) and L is a ligand, or a di-, tri- or tetramer thereof such as [Cp*RuCl]₄.

The ligand L of the Ru-catalyst is optionally selected from COD, NBD, (C₁ to C₁₂ alkyl)CN, or (C₁ to C₁₂ alkyl)₂O, tetrahydrofuran, tetrahydropyran, 1,4-dioxane, substituted pyridines, η-arenes, H₂ and further common readily dissociating dative ligands known in the art.

Preferably, the Ru-catalyst is [Cp*RuCl]₄ (Chloro(pentamethylcyclopentadienyl)ruthenium(II) tetramer). This and related catalysts can also be generated in situ, for example by reaction of [Cp*Ru(MeCN)₃]PF₆ with an appropriate chloride source, such as tetra-n-butylammonium chloride. Alternatively, this and related catalysts can be generated in situ by reduction of an appropriate Ru(+3) precursor, for example the oligomeric complex [Cp*RuCl₂]*ₙ*.

The hydrogen used in this process can be H₂ and any isotopologe thereof, such as HD, or D₂, or any combination of H, D and T including T₂ wherein D represents deuterium and T represents tritium.

If groups such as any two R¹ to R⁶ are defined to optionally form a ring with each other, it is intended that two of said R¹ to R⁶ are forming a ring system with the respective R group on the same carbon atom or with a vicinal R group, or with an even further remote R group. Said ring may include hetero atoms in the ring system such as O, N or S or substituted or protected forms thereof.

In an embodiment of the inventive process for the gem-hydrogenation of an internal alkyne, the compound of Formula (I) is hydrogenated in the presence of a Ru-catalyst in an organic solvent, wherein, in Formula (I), R³ and R⁴ each independently represent (C₁ to C₁₂)alkyl or (hetero)(C₁ to C₁₂)alkyl and R³ and R⁴ optionally form a ring system with each other via the alkyl or the heteroalkyl, and wherein:
R¹ and R² each independently represent
   ∘ hydrogen, optionally with the proviso that at least one of R¹ and R² is not hydrogen;
   ∘ Si(C₁ to C₁₂ alkyl)₃, C(=O)-O-(C₁ to C₁₂ alkyl), or
   ∘ (C₁ to C₁₂)alkyl or hetero(C₁ to C₁₂)alkyl,
      wherein R¹ and R² may form a ring system with each other via the alkyl or the heteroalkyl,
R⁵ and R⁶ each independently represent
   ∘ hydrogen, deuterium,
   ∘ O(C₁ to C₁₂ alkyl), C(=O)-(C₁ to C₁₂ alkyl), C(=O)-O-(C₁ to C₁₂ alkyl),
   ∘ NR^{N1}R^{N2}, wherein R^{N1} represents a protective group selected from an arylsulfonyl group, a (C₁ to C₁₂)alkylsulfonyl group, a (C₁ to C₁₂)alkoxy carbonyl group or -C(=O)-(C₁ to C₁₂ alkyl), and R^{N2} represents a C₁ to C₁₂ alkyl group, or wherein R^{N1} and R^{N2} each represent a C₁ to C₁₂ alkyl group, or
   ∘ (C₁ to C₁₂)alkyl or hetero(C₁ to C₁₂)alkyl,
wherein R⁵ and R⁶ may form a ring system with each other via the alkyl or the heteroalkyl, wherein Q represents O or NR^{N} wherein R^{N} represents a protective group selected from an arylsulfonyl group, a (C₁ to C₁₂)alkylsulfonyl group, a (C₁ to C₁₂)alkoxy carbonyl group or - C(=O)-(C₁ to C₁₂ alkyl).

In another embodiment of the inventive process, the compound of Formula (I) is hydrogenated in the presence of a Ru-catalyst in an organic solvent, wherein, in Formula (I), R³ and R⁴ each independently represent a (C₁ to C₁₂)alkyl or hetero(C₁ to C₁₂)alkyl and R³ and R⁴ optionally form a ring system with each other via the alkyl or the heteroalkyl, Q represents O, R⁵ and R⁶ each independently represent hydrogen, deuterium, (C₁ to C₁₂)alkyl or hetero(C₁ to C₁₂)alkyl and wherein R¹ and R² each independently represent
∘ hydrogen, optionally with the proviso that at least one of R¹ and R² is not hydrogen;
∘ Si(C₁ to C₁₂ alkyl)₃, C(=O)-O-(C₁ to C₁₂ alkyl), or
∘ (C₁ to C₁₂)alkyl or hetero(C₁ to C₁₂)alkyl,
wherein R¹ and R² may form a ring system with each other via the alkyl or the heteroalkyl.

In a further embodiment of the inventive process, the compound of Formula (I) is hydrogenated in the presence of a Ru-catalyst in an organic solvent, wherein, in Formula (I) R³ and R⁴ each independently represent a (C₁ to C₁₂)alkyl or hetero(C₁ to C₁₂)alkyl and R³ and R⁴ optionally form a ring system with each other via the alkyl or the heteroalkyl, Q represents O, R¹ represents hydrogen, R² represents (C₁ to C₁₂)alkyl or hetero(C₁ to C₁₂)alkyl, and wherein R⁵ and R⁶ each independently represent
∘ hydrogen, deuterium,
∘ O(C₁ to C₁₂ alkyl), C(=O)-(C₁ to C₁₂ alkyl), C(=O)-O-(C₁ to C₁₂ alkyl),
∘ NR^{N1}R^{N2}, wherein R^{N1} represents a protective group selected from a arylsulfonyl group, a (C₁ to C₁₂)alkylsulfonyl group, a alkoxy carbonyl group or -C(=O)-(C₁ to C₁₂ alkyl), and R^{N2} represents a C₁ to C₁₂ alkyl group, or wherein R^{N1} and R^{N2} each represent a C₁ to C₁₂ alkyl group, or
∘ (C₁ to C₁₂)alkyl or hetero(C₁ to C₁₂)alkyl,
wherein R⁵ and R⁶ may form a ring system with each other via the alkyl or the heteroalkyl.

In a yet another embodiment of the inventive process, the compound of Formula (I) is hydrogenated in the presence of a Ru-catalyst in an organic solvent, wherein, in Formula (I), R³ and R⁴ each independently represent a (C₁ to C₁₂)alkyl or hetero(C₁ to C₁₂)alkyl and R³ and R⁴ optionally form a ring system with each other via the alkyl or the heteroalkyl, Q represents NR^{N} wherein R^{N} represents a protective group selected from a arylsulfonyl group, a (C₁ to C₁₂)alkylsulfonyl group, a alkoxy carbonyl group or -C(=O)(C₁ to C₁₂ alkyl), and wherein R¹ and R² each independently represent
∘ hydrogen, optionally with the proviso that at least one of R¹ and R² is not hydrogen;
∘ Si(C₁ to C₁₂ alkyl)₃, C(=O)-O-(C₁ to C₁₂ alkyl), or
∘ (C₁ to C₁₂)alkyl or hetero(C₁ to C₁₂)alkyl,
   wherein R¹ and R² may form a ring system with each other via the alkyl or the heteroalkyl, and wherein R⁵ and R⁶ each independently represent
∘ hydrogen, deuterium,
∘ O(C₁ to C₁₂ alkyl), C(=O)-(C₁ to C₁₂ alkyl), C(=O)-O-(C₁ to C₁₂ alkyl),
∘ NR^{N1}R^{N2}, wherein R^{N1} represents a protective group selected from a arylsulfonyl group, a (C₁ to C₁₂)alkylsulfonyl group, a alkoxy carbonyl group or -C(=O)-(C₁ to C₁₂ alkyl), and R^{N2} represents a C₁ to C₁₂ alkyl group, or wherein R^{N1} and R^{N2} each represent a C₁ to C₁₂ alkyl group, or
∘ (C₁ to C₁₂)alkyl or hetero(C₁ to C₁₂)alkyl,
wherein R⁵ and R⁶ may form a ring system with each other via the alkyl or the heteroalkyl.

In a yet further embodiment of the inventive process, the compound of Formula (I) is hydrogenated in the presence of a Ru-catalyst in an organic solvent, wherein, in Formula (I):
- R¹ and R² each independently represent
   ∘ hydrogen, optionally with the proviso that at least one of R¹ and R² is not hydrogen;
   ∘ Si(C₁ to C₁₂ alkyl)₃, C(=O)-O-(C₁ to C₁₂ alkyl), or
   ∘ (C₁ to C₁₂)alkyl or hetero(C₁ to C₁₂)alkyl,
      wherein R¹ and R² may form a ring system with each other via the alkyl or the heteroalkyl,
- R³ and R⁴ each independently represent O(C₁ to C₁₂)alkyl,
- R⁵ represents hydrogen and R⁶ represents hydrogen or (C₁ to C₁₂)alkyl, and
- Q represents O.

In yet another embodiment of the inventive process, the compound of Formula (I) is hydrogenated in the presence of a Ru-catalyst in an organic solvent, wherein, in Formula (I),
Q represents CH₂, CH(C₁ to C₁₂ alkyl), or C(C₁ to C₁₂ alkyl)₂,
R⁴ and R⁵ each independently represent (C₁ to C₁₂)alkyl or hetero(C₁ to C₁₂)alkyl and form a ring system with each other with O or NR^{N} in the ring wherein R^{N} represents a protective group selected from an arylsulfonyl group, a (C₁ to C₁₂)alkylsulfonyl group, a (C₁ to C₁₂)alkoxy carbonyl group or -C(=O)-(C₁ to C₁₂ alkyl),
R³ represents (C₁ to C₁₂)alkyl, hetero(C₁ to C₁₂)alkyl or OSi(C₁ to C₁₂ alkyl)₃,
R⁶ represents hydrogen, deuterium, (C₁ to C₁₂)alkyl or hetero(C₁ to C₁₂)alkyl,, and
R¹ and R² each independently represent:
   ∘ hydrogen, optionally with the proviso that at least one of R¹ and R² is not hydrogen;
   ∘ Si(C₁ to C₁₂ alkyl)₃, C(=O)-O-(C₁ to C₁₂ alkyl), or
   ∘ (C₁ to C₁₂)alkyl or hetero(C₁ to C₁₂)alkyl.

In the inventive process, the solvent for the gem-hydrogenation of an internal alkyne is an organic solvent preferably selected from aprotic apolar organic solvents preferably selected from dichloromethane, chloroform, 1,2-dichloroethane, tetrahydropyran, tetrahydrofuran, ethyl acetate, diethyl ether, di-n-propyl ether, tert-butyl methyl ether, acetone, or mixtures thereof.

In the inventive process, the compound of Formula (I) is hydrogenated in the presence of a Ru-catalyst in an organic solvent under a H₂ partial pressure of 0,1 to 100 atm, preferably 0,5 to 5 bar and more preferred 0,8 atm to 1,5 atm.

The compound of Formula (I) is usually hydrogenated in the presence of a Ru-catalyst in an organic solvent in a temperature range of 0°C to 150°C, preferably in a temperature range of 60°C to 80°C.

The compound of Formula (I) is usually hydrogenated in an organic solvent in the presence of a Ru-catalyst in a molar amount of 1 to 10 mol%, referred to the molar amount of the compound of Formula (I).

The reaction time for the inventive process is not particularly limited and is usually in the range of 30 min to 600 min, preferably 120 min to 240 min.

In the context of the aspects of the present invention, the following definitions are more general terms which are used throughout the present application.

When a range of values is listed, it is intended to encompass each value and sub-range within the range. For example "C₁₋₆" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆.

The term "aliphatic" includes both saturated and unsaturated, straight chain *(i.e.,* unbranched), branched, acyclic, cyclic, or polycyclic aliphatic hydrocarbons, which are optionally substituted with one or more functional groups. As will be appreciated by one of ordinary skill in the art, "aliphatic" is intended herein to include, but is not limited to, alkyl, alkenyl, alkynyl, cycloalkyl, cycloalkenyl, and cycloalkynyl moieties. Thus, the term "alkyl" includes straight, branched and acyclic alkyl groups. An analogous convention applies to other generic terms such as "alkenyl", "alkynyl", and the like. Furthermore, the terms "alkyl", "alkenyl", "alkynyl", and the like encompass both substituted and unsubstituted groups. In certain embodiments, "lower alkyl" is used to indicate those alkyl groups (acyclic, substituted, unsubstituted, branched or unbranched) having 1-6 carbon atoms.

As used herein, "alkyl" refers to a radical of a straight-chain, cyclic or branched saturated hydrocarbon group having from 1 to 12 carbon atoms ("C₁₋₁₂ alkyl"). In some embodiments, an alkyl group has 1 to 10 carbon atoms ("C₁₋₁₀ alkyl"). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). In some embodiments, an alkyl group has 2 to 6 carbon atoms ("C₂₋₆ alkyl"). Examples of C₁₋₆ alkyl groups include methyl (C₁), ethyl (C₂), n-propyl (C₃), isopropyl (C₃), n-butyl (C₄), tert-butyl (C₄), sec-butyl (C₄), iso-butyl (C₄), n-pentyl (C₅), 3-pentanyl (C₅), amyl (C₅), neopentyl (C₅), 3-methyl-2-butanyl (C₅), tertiary amyl (C₅), and n-hexyl (C₆). Additional examples of alkyl groups include n-heptyl (C₇), n-octyl (C₈) and the like. Unless otherwise specified, each instance of an alkyl group is independently unsubstituted (an "unsubstituted alkyl") or substituted (a "substituted alkyl") with one or more substituents. In certain embodiments, the alkyl group is an unsubstituted C₁₋₁₀ alkyl (e.g., -CH₃). In certain embodiments, the alkyl group is a substituted C₁₋₁₀ alkyl.

Aryl" refers to a radical of a monocyclic or polycyclic (*e.g*., bicyclic or tricyclic) 4n+2 aromatic ring system (*e.g*., having 6, 10, or 14 pi electrons shared in a cyclic array) having 6-14 ring carbon atoms and zero heteroatoms provided in the aromatic ring system ("C₆₋₁₄ aryl"). In some embodiments, an aryl group has six ring carbon atoms ("C₆ aryl"; *e.g.*, phenyl). In some embodiments, an aryl group has ten ring carbon atoms ("C₁₀ aryl"; *e.g*., naphthyl such as 1-naphthyl and 2-naphthyl). In some embodiments, an aryl group has fourteen ring carbon atoms ("C₁₄ aryl"; *e.g*., anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Unless otherwise specified, each instance of an aryl group is independently optionally substituted, *i.e.,* unsubstituted (an "unsubstituted aryl") or substituted (a "substituted aryl") with one or more substituents. In certain embodiments, the aryl group is unsubstituted C₆₋₁₄ aryl. In certain embodiments, the aryl group is substituted C₆₋₁₄ aryl.

Alkyl groups, heteroalkyl groups or aryl groups are optionally substituted (*e.g.*, "substituted" or "unsubstituted" alkyl, "substituted" or "unsubstituted" heteroalkyl. In general, the term "substituted", whether preceded by the term "optionally" or not, means that at least one hydrogen present on a group (*e.g*., a carbon or nitrogen atom) is replaced with a permissible substituent, *e.g.,* a substituent which upon substitution results in a stable compound, *e.g.,* a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. For purposes of this invention, heteroatoms such as nitrogen may have hydrogen substituents and/or any suitable substituent as described herein which satisfy the valencies of the heteroatoms and results in the formation of a stable moiety. In certain embodiments, the substituent is a carbon atom substituent. In certain embodiments, the substituent is a nitrogen atom substituent. In certain embodiments, the substituent is an oxygen atom substituent. In certain embodiments, the substituent is a sulfur atom substituent.

Exemplary substituents include, but are not limited to, halogen, -CN, -NO₂, -N₃, -SO₂H, - SO₃H, -OH, -O-alkyl, -N-dialkyl, -SH, -S.alkyl, -C(=O)alkyl,-CO₂H, -CHO. A heterosubstituent may be selected from O, N, S or halogen.

"Halo" or "halogen" refers to fluorine (fluoro, -F), chlorine (chloro, -CI), bromine (bromo, - Br), or iodine (iodo, -I).

If any of R¹ to R⁶ stands for hydrogen, the respective isotopes deuterium and tritium are included in said definition. The same applies to insertions into C-H-bonds, where an insertion into a C-D bond or C-T-bond is also included in said definition.

If any of R¹ to R⁶ stands for hetero(C₁ to C₁₂)alkyl, said definition includes the option that said hetero atom in the meaning of oxygen or nitrogen is bound to the carbon atom bearing the hetero(C₁ to C₁₂)alkyl, and the option that said heteroatom is not directly bound to the to the carbon atom bearing the hetero(C₁ to C₁₂)alkyl, but is contained in the (C₁ to C₁₂)alkyl group. These options also include that said heteroatom is part of a ring structure which is optionally formed by two of R¹ to R⁶.

The invention is further illustrated by the following Figures and Examples. In the Figures, it is shown:
- Figure 1A:: Hydrogenative metathesis for an alkynone
- Figure 1B:: Carbene/alkyne metathesis (CAM),
- Figure 2:: Hydrogenative C-H Insertion
- Figure 3:: Hydrogenative C-H Insertion of Acetals
- Figure 4:: Structural Diversity by Site-Selective C-H Insertion
- Figure 5:: Formation of Pyrrolidine Derivatives by *gem*-Hydrogenation
- Figure 6:: Spirocyclic scaffolds commonly used in medicinal chemistry
- Figure 7:: Exemplary Downstream Functionalization Reagents and conditions: a) (i) O₃, CH₂Cl₂, -78°C; (ii) Me₂S, -78°C → RT, 76%; b) Co(acac)₃ (25 mol%), PhSiH₃, O₂, THF, 69%; c) Fe(acacc)₃, PhSiH₃, methyl acrylate, 1,2-dichloroethane, ethylene glycol, 60°C, 74%; d) (i) O₃, MeOH, -78°C; (ii) TEMPO, FeSO₄·7H₂O, -78°C → RT, 63%; e) (i) O₃, MeOH, -78°C; (ii) TEMPO, FeSO₄·7H₂O, magnesium bis(monoperoxyphthalate) hexahydrate (MMPP), -78°C → RT, 62%; f) (i) O₃, MeOH, -78°C; (ii) PhSSPh, -78°C → 0°C; FeSO₄·7H₂O; g) *m*CPBA, CH₂Cl₂, 45% (over both steps)
- Figure 8:: Hydrogenative C-H Insertion of Orthoesters and Generation of Lactones

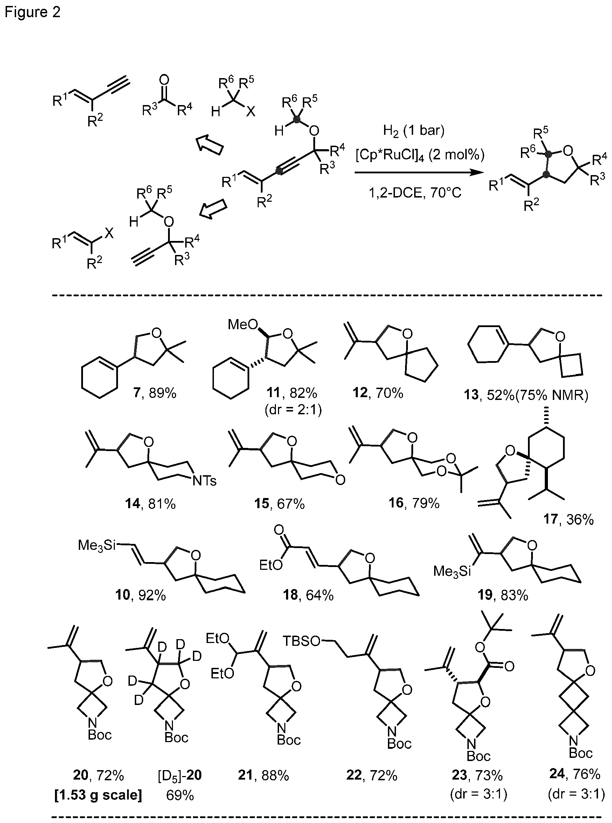

As illustrated in Figure 2, most of these enynes proved amenable to hydrogenative C-H insertion under standard conditions. It is important to note that many of the products shown in Figure 2 would be difficult - if not even impossible - to obtain by the known CAM-based route. Another important aspect is that preconfiguration of the alkene in the substrate allowed compounds such as **10** and **18** to be obtained in isomerically pure form; such control over the configuration is usually not possible by a CAM-based route. An even more significant advantage is the fact that products **12-17** and **19-24** would require hazardous diazomethane as carbene precursor if one were to use CAM for their synthesis, whereas the new route is simple, safe and convenient. For this very reason, the reaction scales well, as illustrated by the preparation of **20**, which was obtained in virtually the same yield independent of whether 23 mg (70%) and 1.53 g (72%) of product were made. Unnecessary to reiterate that this (and any other) compound can also be formed in partially or fully labeled format. Use of perdeutero-methyl iodide and D₂ as two of the cheapest deuterium sources, for example, allowed us to make [D₅]-**20**. Since labelled compounds are of eminent importance in medicinal chemistry and elsewhere, this facile, flexible and, if necessary, scalable entry is arguably significant.

CAM also struggles when it comes to using non-terminal alkynes, since delivery of the primary carbene derived from the diazo derivative is then typically regio-unselective; once again, the new *gem*-hydrogenative approach has no problem in providing access to such products as amply illustrated by Figure 2. Moreover, compounds **7**, **11** and **13** comprising a cyclic alkene moiety would be basically inaccessible via CAM. Although the current study capitalized on insertions into the arguably most challenging primary C-H bonds of methyl ethers, (more activated) secondary and tertiary C-H bonds are also amenable to the reaction (**11**, **23**, Schemes 4, 5).

As illustrated in Figure 3, a noteworthy extension of *gem*-hydrogenation pertains to enynes in which the steering substituent is part of an acetal rather than a simple ether. Figure 3 shows different ways of how the compliance of such substrates could be harnessed. Of arguably highest significance is the ability to form spiroketals from lactones in two straightforward and high-yielding steps via enyne addition/alkylation followed by *gem*-hydrogenative C-H insertion (see compounds **33**, **37**); the inventors are unaware of any precedent for this approach. Importantly, however, the concept is not limited to the formation of spirocycles, since bridged arrays such as **31** and **35** are equally within reach.

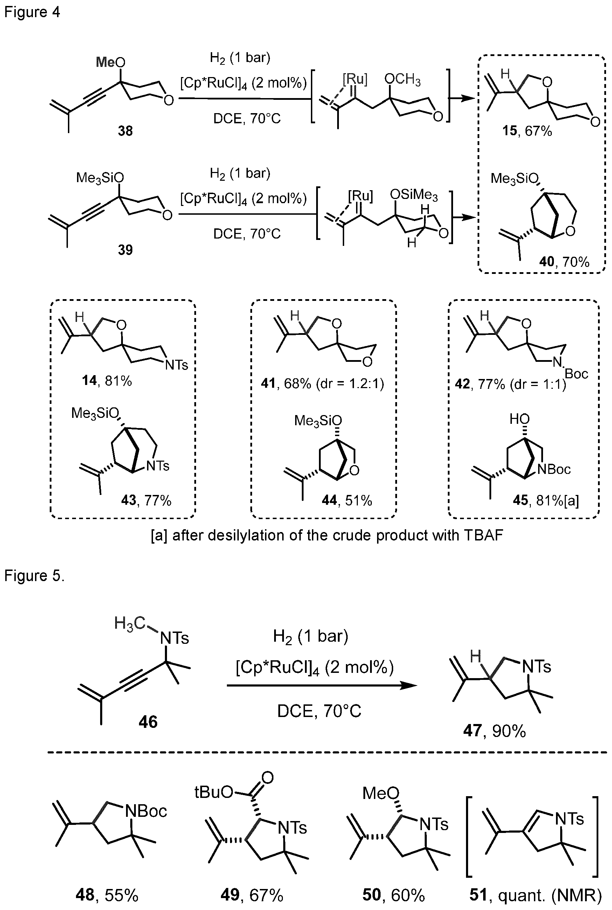

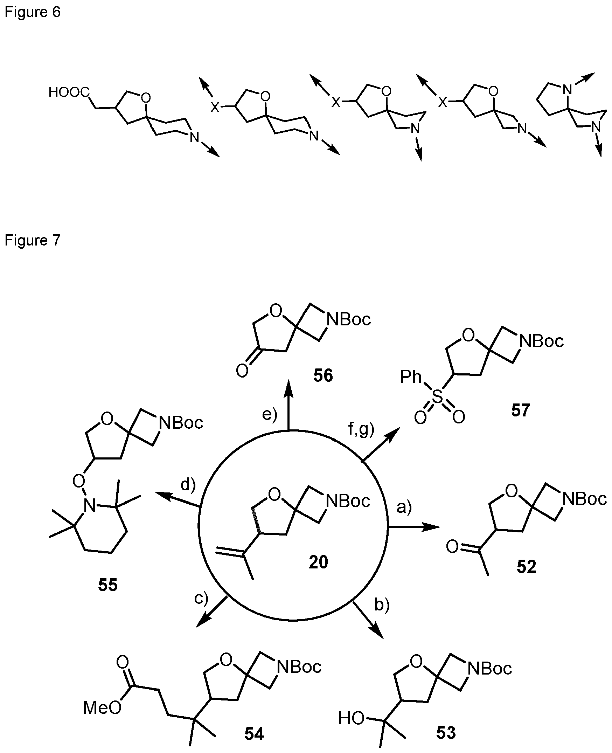

As illustrated in Figure 4, these examples show that the insertion into primary and secondary C-H bonds proceeds with similar efficiency. It was therefore interesting to study whether substrates comprising more than one reactive site are amenable to regioselective C-H insertion. The *gem*-hydrogenation of enyne **38** afforded spirocycle **15** as the only detectable product (Scheme 7). This result is perplexing for the exclusive insertion of the transient ruthenium carbene into the a priori less activated primary C-H bond of the steering -OMe group itself. The analogous reactive intermediate derived from **39**, in contrast, readily engaged the methylene groups of the pre-existing heterocycle into bond formation, giving rise to the bridged bicycle **40** in similar yield. This comparison suggests that the selectivity for the primary C-H bonds is kinetic in origin, which, in turn, implies that the actual C-H insertion step must have a strong steric component to it, likely as a result of the bulky ancillary Cp* ligand. In any case, the ability to produce noticeably different skeletons from a single precursor solely by switching a protecting group is deemed a significant asset. Scaffolds well within reach of this new methodology are prominently featured in contemporary medicinal chemistry as manifested in innumerous patents; the examples shown in Figure 6 are representative.

As shown in Figure 5, the scope of the reaction extends beyond propargyl ethers and acetals. Specifically, *tert*-amide, -carbamate, or -sulfonamide derivatives such as **46** proved well behaved, even though the very nature of the amide group does affect the yield of the resulting pyrrolidine derivative (compare **47**/**48**). The exclusive formation of the cis-configured compounds **49** and **50** is another remarkable feature; actually, **49** can be seen as being remotely related to kainic acid and related neuroexcitatory agents, the structures of which have been edited in countless ways. Hemiaminal **50** is a valuable N-sulfonyliminium ion surrogate; in the absence of external nucleophiles, catalytic HCI converts it into the functionalized 1,3-diene building block **51** in readiness for use in Diels-Alder cycloadditions.

As exemplified in Figure 7 for downstream functionalization, small ring systems in general and spirocyclic scaffolds in particular gained prominence as novel types of building blocks for medicinal chemistry during the last decade. Replacement of traditional flat (hetero)aromatic cores of drug candidates by three-dimensional and sp³-rich templates can be largely beneficial: if properly chosen, they ensure optimal display of attached functionality towards reciprocal groups in the binding site of the targeted biological receptor; moreover, they provide potential advantages with regard to metabolic stability, usually show reduced lipophilicity as compared to (hetero)arenes, open uncommon or even uncharted chemical space, and hence provide many opportunities for chemical innovation and therapeutic advances.

In this context, the ability to provide these building blocks by gem-hydrogenation in isotopically labeled form is deemed a significant asset.

The ease with which *gem*-hydrogenation brings such compounds into reach even on larger scale encouraged the inventors to briefly explore their downstream functionalization. Compound **20** was chosen as the model substrate since its isopropenyl substituent provides a versatile handle (Figure 7) While the cleavage of the double bond by ozonolysis with formation of **52** is an obvious possibility, some other transformations are more involved. Specifically, an iron-catalyzed hydration furnished the tertiary alcohol derivative **53**; rather than trapping the transient radical with oxygen, an intermediate of this type can also be engaged in 1,4-addition reactions to, e. g., ethyl acrylate as illustrated by the formation of **54**. The other entries illustrate the possibility of iron catalyzed dealkenylative oxidation with formation of either the valuable TEMPO-adduct **55** or directly ketone **56**; sulfone **57** further illustrates the structural and functional diversity accessible from a single such platform. In this context, it is of note that compound **56** is a commercially available yet expensive building block, which the new route is able to deliver on scale and, if desirable, in labeled format. Extrapolation of the chemistry shown in Figure 7 to the other (spirocyclic) products containing isopropenyl (or related alkenyl) substituents described above should give access to a multitude of valuable scaffolds for medicinal chemistry and chemical biology.

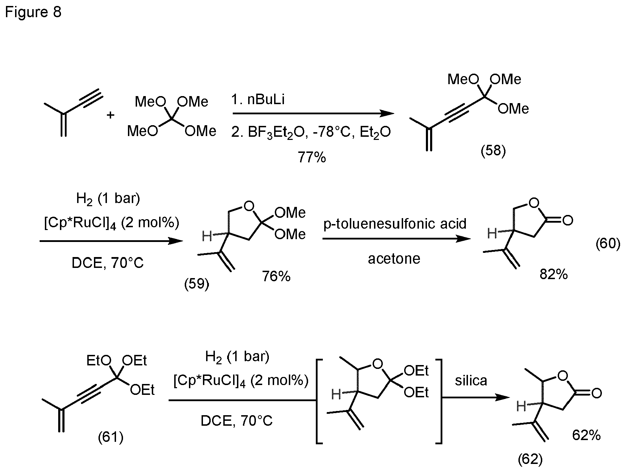

As illustrated in Figure 8, yet another noteworthy extension of *gem*-hydrogenation pertains to enynes in which the steering substituent is part of an orthoester rather than a simple ether or acetal. The resulting ortholactones are difficult to make otherwise; upon hydrolysis, they afford the corresponding lactones.

### Experimental Part

Unless stated otherwise, all reactions were carried out under argon atmosphere in flame-dried Schlenk glassware. The solvents were purified by distillation over the indicated drying agents under argon: THF, Et₂O (Mg/anthracene), hexanes (Na/K), EtOH, MeOH (Mg), 1,2-dichloroethane, CD₂Cl₂, CH₂Cl₂ (CaH₂). DMF, DMSO, MeCN and Et₃N were dried by an absorbtion solvent purification system based on molecular sieves. 1,2-Dichloroethane (DCE), CD₂Cl₂ and CH₂Cl₂ were degassed via freeze-pump-thaw cycles (3 x) and stored over molecular sieves. Flash chromatography: Merck Geduran silica gel 60 (40 - 63 µm). TLCs were stained with KMnO₄, anisaldehyde, or molybdatophosphoric acid (5% in EtOH).

Hydrogen gas (N50, ≥99.999 vol%) was purchased from AirLiquide and was used without further purification. Deuterium gas (99.8 atom% D, 99.995% purity) was purchased from SigmaAldrich. Both H₂ and D₂ were handled with standard balloon techniques.

Unless stated otherwise, all commercially available compounds (abcr, Acros, TCI, Aldrich, Alfa Aesar) were used as received. The ruthenium complex [Cp*RuCl]₄ was prepared according to the literature procedure Organometallics 1990, 9 (6), 1843-1852. The building blocks required for the synthesis of the different substrates were prepared according to the cited literature (see below).

### Preparation of the Substrates

### Non-Commercial Building Blocks

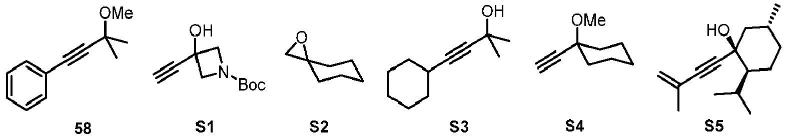

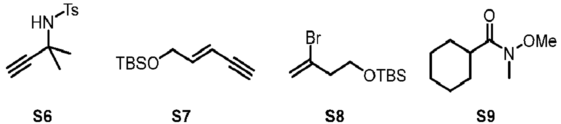

The above shown building blocks were prepared according to literature procedures.

**4-(Cyclohex-1-en-1-yl)-2-methylbut-3-yn-2-ol (S10).** *n*-BuLi (1.6 M in hexanes, 17.7 mL, 28.3 mmol) was slowly added to a solution of 1-ethynylcyclohex-1-ene (3.00 g, 28.3 mmol) in THF (180 mL) at 0 °C. The mixture was stirred for 30 min at 0 °C before acetone (6.2 mL, 84.8 mmol) in THF (15 mL) was slowly introduced. The mixture was stirred for 10 min at 0 °C before sat. NH₄Cl solution (50 mL) and *tert*-butyl methyl ether (150 mL) were added. The layers were separated and the aqueous layer was extracted with *tert*-butyl methyl ether (2 × 150 mL). The combined organic layers were dried over MgSO₄ and the solvent was removed under reduced pressure. The crude product was purified by flash chromatography (silica, hexanes/EtOAc 10:1) to yield the title compound as a colorless oil (4.12 g, 89%).

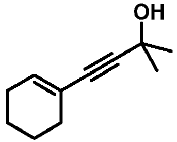

**1-(3-Methylbut-3-en-1-yn-1-yl)cyclopentan-1-ol (S11).** *n*-BuLi (1.6 M in hexanes, 14.2 mL, 22.7 mmol) was slowly added to a solution of 2-methylbut-1-en-3-yne (1.50 g, 22.7 mmol) in THF (150 mL) at 0 °C. The mixture was stirred for 30 min at 0 °C before a solution of cyclopentanone (3.0 mL, 34.0 mmol) in THF (7 mL) was slowly introduced. The mixture was stirred for 10 min at 0 °C before sat. NH₄Cl solution (50 mL) and *tert*-butyl methyl ether (100 mL) were added. The layers were separated and the aqueous layer was extracted with *tert*-butyl methyl ether (2 × 100 mL). The combined organic layers were dried over MgSO₄ and the solvent was removed under reduced pressure. The crude product was purified by flash chromatography (silica, hexanes/EtOAc 10:1) to yield the title compound as a colorless oil (2.47 g, 73%).

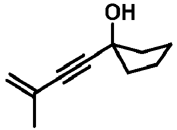

***tert*-Butyl 3-hydroxy-3-(3-methylbut-3-en-1-yn-1-yl)azetidine-1-carboxylate (S12).** *n-* BuLi (1.6 M in hexanes, 5.1 mL, 8.2 mmol) was slowly added to a solution of 2-methylbut-1-en-3-yne (630 mg, 9.5 mmol) in THF (60 mL) at -78 °C. The mixture was stirred for 15 min at -78 °C before 1-Boc-3-azetidinone (1.00 g, 5.8 mmol) in THF (10 mL) was slowly introduced. Stirring was continued at -78 °C for 15 min before the mixture was allowed to warm to -20 °C over 60 min. sat. NH₄Cl solution (40 mL) and EtOAc (40 mL) were added, the layers were separated, and the aqueous phase was extracted with EtOAc (2 × 40 mL). The combined organic layers were dried over MgSO₄ and the solvent was removed under reduced pressure. The crude product was purified by flash chromatography (silica, hexanes/EtOAc, 4:1) to obtain the title compound as white solid (1.15 g, 83 %).

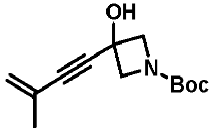

**4-(3-Methylbut-3-en-1-yn-1-yl)-1-tosylpiperidin-4-ol (S13).** Prepared analogously from 1-tosylpiperidin-4-one (253 mg, 2.0 mmol); colorless oil (362 mg, 57 %).

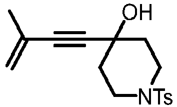

**4-(3-Methylbut-3-en-1-yn-1-yl)tetrahydro-2H-pyran-4-ol (S14).** Prepared analogously from tetrahydro-4*H*-pyran-4-one (417 mg, 4.2 mg); colorless oil (481 mg, 70%).

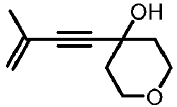

**3-(3-Methylbut-3-en-1-yn-1-yl)tetrahydrofuran-3-ol (S15).** Prepared analogously from tetrahydrofuran-3-on (230 mg, 2.7 mmol); colorless oil (391 mg, 96 %).

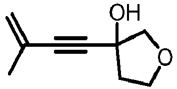

**1-Cyclohexyl-4-methylpent-4-en-2-yn-1-one (S16).** *n*-BuLi (1.6 M in hexanes, 1.7 mL, 2.7 mmol) was slowly added to a solution of 2-methylbut-1-en-3-yne (200 mg, 3.0 mmol) in THF (10 mL) at -78 °C. The mixture was stirred for 15 min at this temperature before a solution of N-methoxy-N-methylcyclohexanecarboxamide (**S9**) (430 mg, 2.5 mmol) in THF (3 mL) was slowly added. Stirring was continued at -78 °C for 15 min and for 60 min at -20 °C. NH₄Cl solution (20 mL) and *tert*-butyl methyl ether (20 mL) were added, the layers were separated, and the aqueous phase was extracted with *tert*-butyl methyl ether (2 × 20 mL). After evaporation of the solvents, the crude product was purified by flash chromatography (silica, hexanes/ *tert*-butyl methyl ether, 98:2) to yield the title compound as a pale yellow oil (377 mg, 85 %).

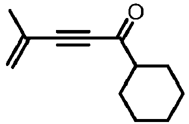

***tert*-Butyl 3-ethynyl-3-methoxyazetidine-1-carboxylate (S17).** A solution of alcohol **S1** (3.05 g, 15.5 mmol) in THF (5 mL) was slowly added to a suspension of NaH (334 mg, 13.9 mmol) in THF (60 mL) and DMF (10 mL) at room temperature. The mixture was stirred for 30 min before a solution of Mel (1.1 mL, 17.0 mmol) in DMF (10 mL) was carefully added. After stirring for antoher 10 min, sat. NH₄Cl solution (25 mL) and EtOAc (100 mL) were introduced and the layers were separated. The aqueous phase was extracted with EtOAc (2 × 100 mL) and the combined organic layers were washed with brine (2 × 15 mL) and dried over MgSO₄. The solvent was evaporated and the residue was purified by flash chromatography (silica, hexanes/EtOAc 10:1 - 5:1) to yield the title compound as a colorless oil (2.85 g, 97% *(wrt. NaH)).*

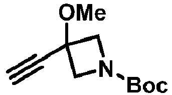

### Sonogashira Coupling.

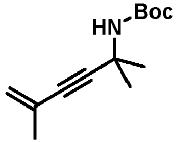

***tert*-Butyl (2,5-dimethylhex-5-en-3-yn-2-yl)carbamate (S18).** NEt₃ (0.57 mL, 4.1 mmol), Cul (52 mg, 0.27 mmol) and Pd(PPh₃)₄ (157 mg, 0.14 mmol) were added to a stirred solution of 2-bromoprop-1-ene (500 mg, 4.1 mmol) and *tert*-butyl (2-methylbut-3-yn-2-yl)carbamate (250 mg, 1.36 mmol) in DMF (7 mL). The mixture was stirred for 2 h at room temperature before sat. NH₄Cl solution (7 mL) and *tert*-butyl methyl ether (15 mL) were introduced. The layers were separated and the aqueous phase was extracted with *tert*-butyl methyl ether (2 × 25 mL). The combined organic layers were washed with sat. NaCl solution (2 × 10 mL) and dried over MgSO₄. The solvent was removed under reduced pressure and the residue was purified by flash chromatography (silica, hexanes/EtOAc 20:1 - 10:1) to yield the title compound as a colorless oil (165 mg, 54%).

**N-(2,5-Dimethylhex-5-en-3-yn-2-yl)-4-methylbenzenesulfonamide (S19).** Prepared analogously from propargylamine **S6** (2.00 g, 8.43 mmol) as a pale yellow oil (1.85 g, 79%).

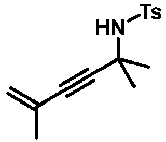

### Substrates for gem-Hydrogenation/C-H Insertion

### Via Alkylation

**1-(3-Methoxy-3-methylbut-1-yn-1-yl)cyclohex-1-ene (5).** A solution of alcohol **S10** (300 mg, 1.8 mmol) in THF (1 mL) was added to a stirred suspension of NaH (109 mg, 4.6 mmol) in THF (7 mL) at 0 °C. Stirring was continued for 30 min at room temperature before Mel (0.57 mL, 9.1 mmol) was introduced. After stirring for another 1 h, sat. NH₄Cl solution (3 mL), water (6 mL) and *tert*-butyl methyl ether (30 mL) were added and the layers separated. The aqueous phase was extracted with *tert*-butyl methyl ether (2 × 30 mL) and the combined organic layers were washed with brine and dried over MgSO₄. The solvent was removed under reduced pressure and the crude product was purified by flash chromatography (silica, hexanes/EtOAc 1:0 - 40:1) to give the title compound as a colorless oil (210 mg, 64%).

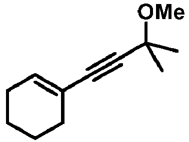

**(3-(Methoxy-[D])-3-methylbut-1-yn-1-yl)cyclohex-1-ene ([Di]-5).** Prepared analogously using mono-deutero-methyl iodide; colorless oil (287 mg, 88%).

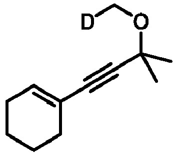

**1-(3-(Methoxy-[D₂])-3-methylbut-1-yn-1-yl)cyclohex-1-ene ([D₂]-5).** Prepared analogously using dideutero-methyl iodide; colorless oil (282 mg, 86%).

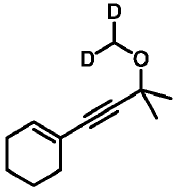

**1-(3-([D₃]-Methoxy)-3-methylbut-1-yn-1-yl)cyclohex-1-ene ([D₃]-5).** Prepred analogously using CD₃I; colorless oil (250 mg, 76%).

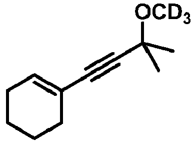

**1-Methoxy-1-(3-methylbut-3-en-1-yn-1-yl)cyclopentane (S20).** A solution of alcohol **S11** (280 mg, 1.9 mmol) in THF (1 mL) was added to a stirred suspension of NaH (112 mg, 4.7 mmol) in THF (7 mL) at 0 °C. The mixture was stirred for 30 min at room temperature before Mel (0.58 mL, 9.3 mmol) was introduced. After stirring for 1 h at room temperature, sat. NH₄Cl solution (3 mL), water (6 mL) and *tert*-butyl methyl ether (30 mL) were added and the layers separated. The aqueous phase was extracted with *tert*-butyl methyl ether (2 × 30 mL) and the combined organic layers were washed with brine and dried over MgSO₄. The solvent was removed under reduced pressure and the crude product was purified by flash chromatography (silica, hexanes/EtOAc 1:0 - 40:1) to give the title compound as a colorless oil (225 mg, 73%).

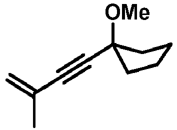

**1-((1-Methoxycyclobutyl)ethynyl)cyclohex-1-ene (S21).** Prepared analogously from cyclobutanone and 1-ethynylcyclohex-1-ene; colorless oil (230 mg, 94%).

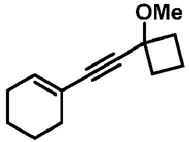

**(1S,2S,5R)-2-Isopropyl-1-methoxy-5-methyl-1-(3-methylbut-3-en-1-yn-1-yl)cyclohexane (S22).** A solution of alcohol **S5** (175 mg, 0.79 mmol) in DMF (1 mL) was added to a stirred suspension of NaH (48 mg, 2.0 mmol) in DMF (4 mL) at room temperature. The mixture was stirred for 20 min before Mel (0.25 mL, 1.6 mmol) was introduced. After stirring for another 2 h, sat. NH₄Cl solution (6 mL), water (3 mL) and *tert*-butyl methyl ether (15 mL) were added and the layers separated. The aqueous phase was extracted with *tert-butyl* methyl ether (2 × 30 mL) and the combined organic layers were washed with brine (3 × 10 mL) and dried over MgSO₄. The solvent was removed under reduced pressure and the crude product was purified by flash chromatography (silica, hexanes/EtOAc 1:0 - 100:1) to give the title compound as a colorless oil (25.9 mg, 14%).

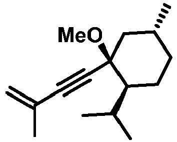

**(3-Methoxy-3-methylbut-1-yn-1-yl)cyclohexane (60).** A solution of alcohol **S3** (300 mg, 1.80 mmol) in THF (1 mL) was added to a stirred suspension of NaH (108 mg, 4.51 mmol) in THF (7 mL) at 0 °C. The mixture was stirred for 30 min at room temperature before Mel (0.56 mL, 9.0 mmol) was introduced. After stirring for another 1 h, sat. NH₄Cl solution (3 mL), water (6 mL) and *tert*-butyl methyl ether (30 mL) were added and the layers separated. The aqueous phase was extracted with *tert*-butyl methyl ether (2 × 30 mL) and the combined organic layers were washed with brine and dried over MgSO₄. The solvent was removed under reduced pressure and the crude product was purified by flash chromatography (silica, hexanes/EtOAc 1:0 - 40:1) to give the title compound as a colorless oil (268 mg, 82%).

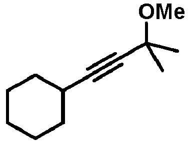

**1-(3-(Methoxymethoxy)-3-methylbut-1-yn-1-yl)cyclohex-1-ene (S23).** A solution of alcohol **S10** (300 mg, 1.8 mmol) in THF (1 mL) was added to a stirred suspension of NaH (88 mg, 3.7 mmol) in THF (7 mL) at 0 °C. The mixture was stirred for 30 min at room temperature before MOMCI (0.28 mL, 3.7 mmol) was slowly introduced at 0 °C. After stirring for 18 h at room temperature, sat. NH₄Cl solution (3 mL), water (6 mL) and *tert*-butyl methyl ether (30 mL) were added and the layers separated. The aqueous phase was extracted with *tert*-butyl methyl ether (2 × 30 mL) and the combined organic layers were washed with brine and dried over MgSO₄. The solvent was removed under reduced pressure and the crude product was purified by flash chromatography (silica, hexanes/EtOAc 1:0 - 30:1) to give the title compound as a colorless oil (169 mg, 44%).

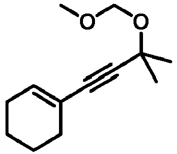

***tert*-Butyl 3-(2-(*tert*-butoxy)-2-oxoethoxy)-3-(3-methylbut-3-en-1-yn-1-yl)azetidine-1-carboxylate (S24).** A solution of alcohol **S12** (200 mg, 0.84 mmol) in DMF (1 mL) was added to a stirred suspension of NaH (51 mg, 2.1 mmol) in DMF (4 mL) at room temperature. The mixture was stirred for 20 min before *tert*-butyl 2-bromoacetate (0.31 mL, 2.1 mmol) was introduced. After stirring for 15 min at room temperature, sat. NH₄Cl solution (3 mL), water (3 mL) and EtOAc (15 mL) were added and the layers were separated. The aqueous phase was extracted with EtOAc (2 × 15 mL) and the combined organic layers were washed with brine (2 × 10 mL) and dried over MgSO₄. The solvent was removed under reduced pressure and the crude product was purified by flash chromatography (silica, hexanes/EtOAc 10:1) to give the title compound as a colorless oil (271 mg, 91%).

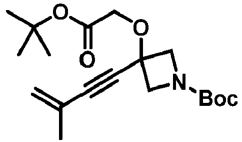

### Via One-Pot Alkynylation/O-Alkylation

***tert*-Butyl 3-methoxy-3-(3-methylbut-3-en-1-yn-1-yl)azetidine-1-carboxylate (S25)**. *n-*BuLi (1.6 M in hexanes, 1.07 mL, 1.72 mmol) was added to a stirred solution of 2-methyl-1-buten-3-yne (122 mg, 1.84 mmol) in THF (7.4 mL) at -78 °C under argon and the resulting mixture was stirred for 15 min at that temperature. A solution of 1-Boc-3-azetidinone (210 mg, 1.23 mmol) in THF (2.0 mL) was slowly added and the mixture was stirred for 15 min before it was allowed to warm to -20 °C over the course of 1 h. A solution of Mel (0.31 mL, 4.91 mmol) in DMSO (4.8 mL) was added and the mixture was allowed to reach room temperature within 1 h. The reaction was quenched by addition of sat. NH₄Cl solution (6 mL) and water (6 mL) and the biphasic mixture was extracted with EtOAc (3 × 30 mL). The combined organic layers were washed with brine (2 × 10 mL), dried over MgSO₄ and the solvent was removed under reduced pressure. The crude product was purified by flash chromatography (silica, hexanes/EtOAc 20:1 - 10:1) to obtain the title compound as a colorless oil (270 mg, 88%). *When performed on larger scale, the yield of product was even higher* (2.16 g, 92%).

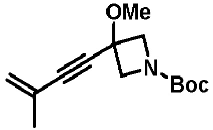

***tert*-Butyl 3-(methoxy-[D₃])-3-(3-methylbut-3-en-1-yn-1-yl)azetidine-1-carboxylate ([D₃]-S25**). Prepared analogously from 1-Boc-3-azetidinone and CD₃I; colorless oil (276 mg, 88%).

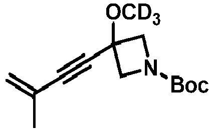

**4-Methoxy-4-(3-methylbut-3-en-1-yn-1-yl)-1-tosylpiperidine (S26).** Prepared analogously from 1-tosylpiperidin-4-one; colorless solid (332 mg, 79%).

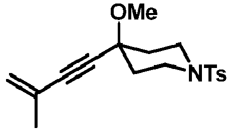

**4-Methoxy-4-(3-methylbut-3-en-1-yn-1-yl)tetrahydro-2H-pyran (38).** Prepared analogously from tetrahydro-4*H*-pyran-4-one; colorless oil (164 mg, 73%).

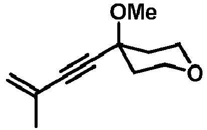

**5-Methoxy-2,2-dimethyl-5-(3-methylbut-3-en-1-yn-1-yl)-1,3-dioxane (S27).** Prepared analogously from 2,2-dimethyl-1,3-dioxan-5-one; pale yellow oil (198 mg, 76%).

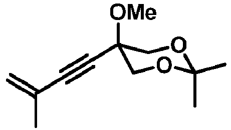

***tert*-Butyl 6-Methoxy-6-(3-methylbut-3-en-1-yn-1-y))-2-azaspiro[3.3]heptane-2-carboxylate (S28).** Prepared analogously from the corresponding spiroketone; pale yellow oil (336 mg, 93%).

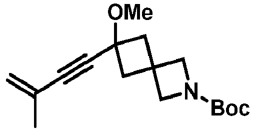

***tert*-Butyl (*E*)-3-(5-((*tert*-butyldimethylsilyl)oxy)pent-3-en-1-yn-1-yl)-3-methoxyazet-idine-1-carboxylate (S29).** Prepared analogously from 1-Boc-3-azetidinone and (*E*)-*tert*-butyldimethyl(pent-2-en-4-yn-1-yloxy)silane (**S7**); colorless oil (400 mg, 89 %).

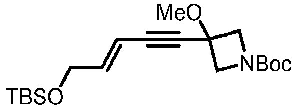

**Preparation of *tert*-Butyl 3-methoxy-3-(3-methylbut-3-en-1-yn-1-yl)pyrrolidine-1-carboxylate (S30).** Prepared analogously from N-Boc-3-pyrrolidinone (185 mg, 1.0 mmol); colorless oil (127 mg, 48 %).

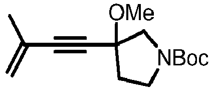

**3-Methoxy-3-(3-methylbut-3-en-1-yn-1-yl)tetrahydrofuran (S31).** Prepared analogously from tetrahydrofuran-3-one (225 mg, 2.6 mmol); colorless oil (329 mg, 76 %).

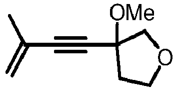

**1-Methoxy-1-(prop-2-yn-1-yl)cyclohexane (9).** Oxirane **S2** (1.29 g, 11.5 mmol) was slowly added to a stirred suspension of lithium acetylide ethylenediamine complex (1.38 g, 15.0 mmol) in DMSO (12 mL) at 0 °C. The mixture was allowed to reach room temperature and stirring was continued for 5 d before sat. NH₄Cl solution (20 mL) and *tert*-butyl methyl ether (50 mL) were added. The aqueous phase was extracted with *tert*-butyl methyl ether (2 × 100 mL) and the combined organic layers were washed with HCI (2 M, 25 mL), sat. CuSO₄ solution (25 mL) and brine, and were then dried over MgSO₄. The solvent was removed under reduced pressure and the crude product was purified by flash chromatography (silica, hexanes/EtOAc 20:1 - 10:1) to give the corresponding homopropargylic alcohol (861 mg, 54%). A solution of this homopropargylic alcohol (409 mg, 2.96 mmol) in THF (1 mL) was added to a stirred suspension of NaH (63 mg, 2.66 mmol) in THF (4 mL) at 0 °C. The mixture was stirred for 30 min at room temperature before Mel (0.20 mL, 3.25 mmol) was introduced. After stirring for another 2 h at room temperature, sat. NH₄Cl solution (3 mL), water (3 mL) and *tert*-butyl methyl ether (30 mL) were introduced and the layers separated. The aqueous phase was extracted with *tert*-butyl methyl ether (2 × 30 mL) and the combined organic layers were washed with brine and dried over MgSO₄. The solvent was removed under reduced pressure and the crude product was purified by flash chromatography (silica, hexanes/EtOAc 1:0 - 50:1) to give the title compound as a colorless oil (196 mg, 48%).

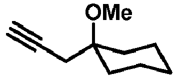

### Via Sonogashira Coupling

**(*E*)-(4-(1-Methoxycyclohexyl)but-1-en-3-yn-1-yl)trimethylsilane (8).** *i*-Pr₂NEt (0.97 mL, 5.6 mmol), Cul (106 mg, 0.56 mmol) and Pd(PPh₃)₂Cl₂ (196 mg, 0.28 mmol) were added to a stirred solution of (*E*)-(2-bromov-inyl)trimethylsilane (500 mg, 2.79 mmol) and alkyne **S4** (500 mg, 3.63 mmol) in DMF (14 mL). The mixture was stirred for 1 h at room temperature before sat. NH₄Cl solution (15 mL) and *tert*-butyl methyl ether (30 mL) were introduced. The layers were separated and the aqueous phase was extracted with *tert*-butyl methyl ether (2 × 50 mL). The combined organic layers were washed with sat. NaCl solution (3 × 10 mL) and dried over MgSO₄. The solvent was removed under reduced pressure and the residue was purified by flash chromatography (silica, hexanes/EtOAc 1:0 - 100:1 - 50:1) to yield the title compound as a pale orange oil (288 mg, 44%).

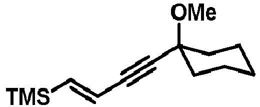

**Ethyl (*E*)-5-(1-methoxycyclohexyl)pent-2-en-4-ynoate (S32).** *i*-Pr₂NEt (0.92 mL, 5.3 mmol), Cul (101 mg, 0.53 mmol) and Pd(PPh₃)₂Cl₂ (185 mg, 0.26 mmol) were added to a stirred solution of ethyl (*E*)-3-bromoacrylate (500 mg, 2.64 mmol) and alkyne **S4** (434 mg, 3.14 mmol) in DMF (13 mL). The mixture was stirred for 1 h at room temperature before sat. NH₄Cl solution (15 mL) and *tert*-butyl methyl ether (30 mL) were introduced. The layers were separated and the aqueous phase was extracted with *tert*-butyl methyl ether (2 × 50 mL). The combined organic layers were washed with sat. NaCl solution (3 × 10 mL) and dried over MgSO₄. The solvent was removed under reduced pressure and the residue was purified by flash chromatography (silica, hexanes/EtOAc 1:0 - 40:1) to yield the title compound as a colorless oil (376 mg, 60%).

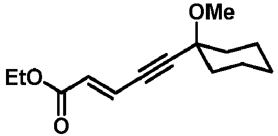

**(4-(1-Methoxycyclohexyl)but-1-en-3-yn-2-yl)trimethylsilane (S33).** *i*-Pr₂NEt (0.97 mL, 5.6 mmol), Cul (106 mg, 0.56 mmol) and Pd(PPh₃)₂Cl₂ (196 mg, 0.28 mmol) were added to a stirred solution of (1-bromovinyl)trimethylsilane (500 mg, 2.79 mmol) and alkyne **S4** (500 mg, 3.63 mmol) in DMF (14 mL). The mixture was stirred for 1 h at room temperature before sat. NH₄Cl solution (15 mL) and *tert*-butyl methyl ether (30 mL) were introduced. The layers were separated and the aqueous phase was extracted with *tert*-butyl methyl ether (2 × 50 mL). The combined organic layers were washed with sat. NaCl solution (3 × 10 mL) and dried over MgSO₄. The solvent was removed under reduced pressure and the residue was purified by flash chromatography (silica, hexanes/EtOAc 1:0 -100:1 - 50:1) and HPLC to yield the title compound as a colorless oil (120 mg, 18%).

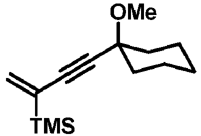

**tert-Butyl 3-(3-(diethoxymethyl)but-3-en-1-yn-1-yl)-3-methoxyazetidine-1-carboxylate (S34).** Prepared analogously from *tert*-butyl 3-ethynyl-3-methox-yazetidine-1-carboxylate (211 mg, 1.0 mmol) (**S17**) and 2-bromo-propenal diethyl acetal (250 mg, 1.3 mmol), colorless oil (69.2 mg, 22%).

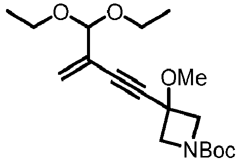

***tert*-Butyl 3-(5-((*tert*-butyidimethylsilyl)oxy)-3-methylenepent-1-yn-1-yl)-3-methox-yazetidine-1-carboxylate (S35).** A solution of *tert*-butyl 3-ethynyl-3-methoxyazetidine-1-carboxylate (**S17**) (220 mg, 1.1 mmol) and ((3-bro-mobut-3-en-1-yl)oxy)(*tert*-butyl)dimethylsilane (**S8**) (325 mg, 1.2 mmol) in THF (2.0 mmol) was added to a solution of Cul (4.9 mg, 0.03 mmol) and Pd(PPh₃)₄ (3.6 mg, 0.003 mmol) in NEt₃ (0.5 mL). The mixture was stirred at ambient temperature until TLC indicated full conversion. The mixture was filtered through a plug of Florisil, eluting with *tert*-butyl methyl ether. The combined filtrates were successively washed with HCl (0.1 M, 15 mL) and NaHCO₃ (15 mL), dried over MgSO₄, and evaporated. Purification of the crude product by flash chromatography (silica, hexanes/ *tert-*butyl methyl ether, 85:15) gave the title compound as a colorless oil (76.7 mg, 19 %).

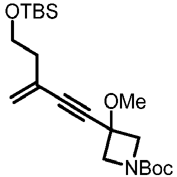

***tert*-Butyl 3-methoxy-3-(3-(trifluoromethyl)but-3-en-1-yn-1-yl)azetidine-1-carboxylate (S36).** Prepared analogously from alkyne **S17** (211 mg, 1.0 mmol) and 2-bromo-3,3,3-trifluoro-1-propene (227.0 mg, 1.3 mmol); pale yellow oil (217 mg, 72 %).

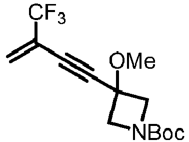

***tert*-Butyl 4-((1-(tert-butoxycarbonyl)-3-methoxyazetidin-3-yl)ethynyl)-3,6-dihydropyridine-1(2*H*)- carboxylate (S37).** Prepared analogously from alkyne **S17** (217 mg, 1.0 mmol) and N-Boc-4-trifluoro-methanesulfonyloxy-3,6-dihydro-2*H*-pyridine (398.0 mg, 1.2 mmol); colorless oil (400.0 mg, 99%).

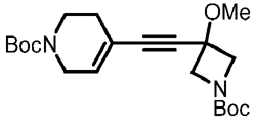

***tert*-Butyl 3-methoxy-3-((5-oxo-2,5-dihydrofuran-3-yl)ethynyl)azetidine-1-carboxylate(S38).** Prepared analogously from alkyne **S17** (209 mg, 0.099 mmol) and 4-bromofuran-2(5*H*)-one (209.5 mg, 1.3 mmol); colorless oil (255 mg, 80 %).

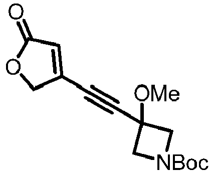

***tert*-butyl 3-methoxy-3-(3-((trimethylsilyl)methyl)but-3-en-1-yn-1-yl)azetidine-1-carboxylate (S39).** Prepared analogously from alkyne **S17** (209 mg, 1.0 mmol) and 2-bromo-allyl-trimethylsilane (272 mg, 1.3 mmol); colorless oil (255 mg, 80 %).

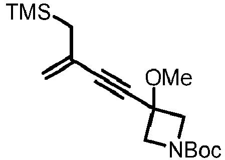

### Acetal Series

**2-Cyclohexyl-2-(3-methylbut-3-en-1-yn-1-yl)-1,3-dioxane1-ciclohexyl (30).** Trimethyl orthoformate (0.11 mL, 1.0 mmol) and *p*-TsOH·H₂O (6.0 mg, 0.08 mmol) were added to a solution of 4-methylpent-4-en-2- yn-1-one (**S16**) (150 mg, 0.85 mmol) in 1,3-propanediol (4.0 mL). The mixture was stirred for 24 h before the reaction was quenched with sat. NaHCO₃ (15 mL) and *tert*-butyl methyl ether (15 mL). The aqueous phase was extracted with *tert-*butyl methyl ether (2 × 15 mL), and the combined organic layers were washed with brine (10 mL) and dried over MgSO₄. The solvent was removed under reduced pressure to yield the title compound as pale yellow oil (169 mg, 85 %).

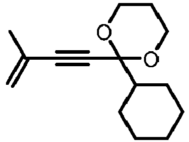

**(1,1-Dimethoxy-4-methylpent-4-en-2-yn-1-yl)cyclohexane (S40).** Trimethyl orthoformate (0.47 mL, 4.3 mmol) was added to a solution of 1-cyclohexyl- 4-methylpent-4-en-2-yn-1-one **(S16)** (150 mg, 0.85 mmol) in MeOH (3.0 mL) at 0 °C., followed by *p*-TsOH·H₂O (6.0 mg, 0.08 mmol). Once GC/MS indicated full conversion, sat. NaHCO₃ (15 mL) and *tert*-butyl methyl ether (15 mL) were introduced, the aqueous phase was extracted with *tert*-butyl methyl ether (2 × 15 mL), and the combined organic phases were washed with brine (10 mL) and dried over MgSO₄. The solvent was removed under reduced pressure to yield the title compound as pale yellow oil (189 mg, quant).

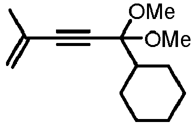

**2-Cyclohexyl-2-(3-methylbut-3-en-1-yn-1-yl)-1,3-dioxolane (S41).** Trimethyl orthoformate (0.11 mL, 1.0 mmol) and *p*-TsOH·H₂O (6.0 mg, 0.08 mmol) were added to a solution of 1-cyclohexyl- 4-methylpent-4-en-2-yn-1-one (**S16**) (150 mg, 0.85 mmol) in ethylene glycol (4.0 mL). The mixture was stirred for 24 h before sat. NaHCO₃ (15 mL) and *tert-butyl* methyl ether (15 mL) were added. The layers were separated, the aqueous phase was extracted with *tert-butyl* methyl ether (2 × 15 mL), and the combined organic layers were washed with brine (10 mL) and dried over MgSO₄. The solvent was removed under reduced pressure to yield the title compound as pale yellow oil (167 mg, 89 %).

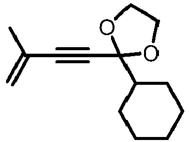

**2-Methoxy-2-(3-methylbut-3-en-1-yn-1-yl)tetrahydrofuran (32).** *n*-BuLi (1.6 M in hexanes, 4.25 mL, 6.81 mmol) was added to a stirred solution of 2-methyl-1-buten-3-yne (500 mg, 7.56 mmol) in THF (30 mL) at -78 °C under argon and was stirred for 30 min at that temperature. BF₃·OEt₂ (1 M in Et₂O, 8.3 mL, 8.3 mmol) was added and the mixture stirred for 30 min at -78 °C before γ-butyrolactone (0.70 mL, 9.1 mmol) was slowly introduced. The mixture was allowed to warm to room temperature and stirring was continued for 18 h before sat. NH₄Cl solution (6 mL) and water (6 mL) were added at 0 °C. The biphasic mixture was extracted with EtOAc (3 × 75mL) and the combined organic layers were washed with brine and dried over MgSO₄. The solvent was removed under reduced pressure and the crude product was purified by flash chromatography (silica, hexanes/EtOAc 2:1 - 3:2 - 1:1) to yield a mixture of the corresponding γ-hydroxyketone, its lactol and its butyric acid ester (692 mg, -60%). This mixture (200 mg, ∼1.3 mmol) was dissolved in MeOH (5 mL) and *p*-TsOH·H₂O (25 mg, 0.13 mmol) was added at room temperature. The mixture was stirred for 18 h before sat. NaHCO₃ solution (3 mL) was introduced. The mixture was extracted with *tert*-butyl methyl ether (3 × 20 mL) and the combined organic layers were washed with brine and dried over MgSO₄. The solvent was removed under reduced pressure and the crude product was purified by flash chromatography (silica, hexanes/EtOAc 20:1) to yield the title product as a colorless oil (126 mg, 58%).

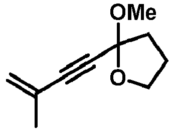

**2-Isopropoxy-2-(3-methylbut-3-en-1-yn-1-yl)tetrahydrofuran (S42).** Prepared analogously using *i*PrOH instead of MeOH (101 mg, 34%).

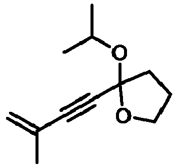

**2-((4-(Cyclohex-1-en-1-yl)-2-methylbut-3-yn-2-yl)oxy)tetrahydro-2H-pyran (S43).** 3,4-Dihydro-2*H*-pyran (0.14 mL, 1.6 mmol) and toluenesulfonic acid monohydrate (11 mg, 0.06 mmol) were added to a solution of 4-(cyclohex-1-en-1-yl)-2-methylbut-3-yn-2-ol (200 mg, 1.2 mmol) (**S10**) in CH₂Cl₂ (2.5 mL) at 0°C and the resulting mixture was stirred at this temperature overnight. sat. NaHCO₃ (15 mL) and *tert*-butyl methyl ether (15 mL) were introduced, the layers were separated, the aqueous phase was extracted with *tert*-butyl methyl ether (2 × 15 mL), and the combined organic layers were washed with brine (10 mL) and dried over MgSO₄. The solvent was removed under reduced pressure and the crude material purified by flash chromatography (silica, hexanes/ *tert*-butyl methyl ether, 98:2) to give the title compound as a colorless oil (90.2 mg, 30%).

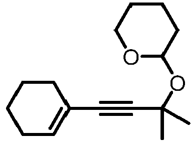

### Silylether Derivatives

**Trimethyl((4-(3-methylbut-3-en-1-yn-1-yl)tetrahydro-2H-pyran-4-yl)oxy)silane (39).** 1*H*-Imidazol (70 mg, 1.0 mmol) was added in one portion to a solution of 4-(3-methylbut-3-en-1-yn-1-yl)tetrahydro-2*H*-pyran-4-ol (**S14**) (150 mg, 0.90 mmol) in CH₂Cl₂ (3.0 mL) and the resulting mixture was stirred for 15 min. TMSCI (0.12 mL, 0.96 mmol) was added and stirring continued for 2 h. The reaction was quenched with water (20 mL) and the mixture diluted with 20 mL of *tert-butyl* methyl ether (20 mL). The layers were separated and the aqueous phase was extracted with *tert-*butyl methyl ether (2 × 20 mL). The combined organic layers were washed with brine and dried over MgSO₄, the solvent was removed under reduced pressure, and the crude product was purified by flash chromatography (silica, hexanes/Et₂O 98:2) to give the title compound as a colorless oil (211 mg, 92 %).

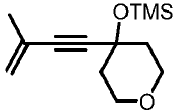

**4-(3-Methylbut-3-en-1-yn-1-yl)-1-tosyl-4-((trimethylsilyl)oxy)piperidine (S44).** Prepared analogously from from 4-(3-methylbut-3-en-1-yn-1-yl)-1-tosylpi-peridin-4-ol (**S13**) (200 mg, 0.63 mmol); colorless oil (245 mg, 99 %).

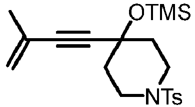

**Trimethyl((3-(3-methylbut-3-en-1-yn-1-yl)tetrahydrofuran-3-yl)oxy)silane (S45).** Prepared analogously from from 3-(3-methylbut-3-en-1-yn-1-yl)tetrahydrofuran-3-ol (**S15**) (200 mg, 1.31 mmol), colorless oil (200 mg, 68 %).

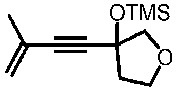

***tert*-Butyl 3-(3-methylbut-3-en-1-yn-1-yl)-3-((trimethylsilyl)oxy)pyrrolidine-1-carboxylate (S46).** *n*-BuLi (1.6 M in hexanes, 0.88 mL, 1.4 mmol) was slowly added to a solution of 2-methylbut-1-en-3-yne (153 mg, 1.6 mmol) in THF (6.0 mL) at-78 °C. The mixture was stirred for 15 min at -78 °C before a solution of N-Boc-3-pyrrolidinone (190 mg, 1.0 mmol) in THF (2.0 mL) was slowly added. The mixture was stirred at -78 °C for 15 min and then warmed to -20 °C over 60 min. sat. NH₄Cl solution (40 mL) and EtOAc (40 mL) were introduced, the layers were separated, and the aqueous phase was extracted with EtOAc (2 × 40 mL). The combined organic layers were dried over MgSO₄ and the solvent was removed under reduced pressure. The crude product was purified by flash chromatography (silica, hexanes/EtOAc, 4:1) to yield the corresponding *tert*-alcohol in slightly impure form (200 mg, ∼70 %). 1*H*-Imidazol (63 mg, 0.93 mmol) was added to a solution of this compound (200 mg, ~0.72 mmol) in CH₂Cl₂ (3.0 mL). After stirring for 15 min, TMSCI (0.11 mL, 0.86 mmol) was introduced and the resulting mixture was stirred for 2 h. The reaction was quenched with water (20 mL) and *tert*-butyl methyl ether (20 mL), the layers were separated, and the aqueous phase was extracted with *tert*-butyl methyl ether (2 × 20 mL). The combined organic layers were washed with brine and dried over MgSO₄. The solvent was removed under reduced pressure and the crude material was purified by flash chromatography (silica, hexanes/ *tert*-butyl methyl ether, 98:2) to give the title compound as a colorless oil (150 mg, 65 %).

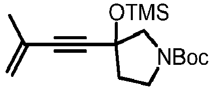

### Propargyl Amine Derivatives

***tert*-Butyl (2,5-dimethylhex-5-en-3-yn-2-yl)(methyl)carbamate (S47).** A solution of carbamate **S18** (160 mg, 0.72 mmol) in DMF (1 mL) was added to a stirred suspension of NaH (26 mg, 1.1 mmol) in DMF (3 mL) at room temperature. The mixture was stirred for 30 min before Mel (0.10 mL, 1.6 mmol) was introduced. After stirring for 1 h at room temperature, sat. NH₄Cl solution (3 mL), water (3 mL) and *tert*-butyl methyl ether (15 mL) were added and the layers separated. The aqueous phase was extracted with *tert*-butyl methyl ether (2 × 15 mL) and the combined organic layers were washed with brine (3 × 10 mL) and dried over MgSO₄. The solvent was removed under reduced pressure and the crude product was purified by flash chromatography (silica, hexanes/EtOAc 40:1 - 20:1) to give the title compound as a colorless oil (148 mg, 87%).

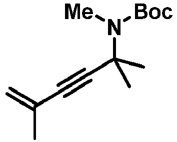

**N-(2,5-Dimethylhex-5-en-3-yn-2-yl)-N,4-dimethylbenzenesulfonamide (46).** Prepared analogously from enyne **S19** (300 mg, 1.08 mmol) as a colorless oil (270 mg, 86%).

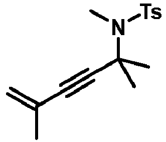

***tert*-Butyl N-(2,5-dimethylhex-5-en-3-yn-2-yl)-N-tosylglycinate (S48).** Sodium hydride (26.0 mg, 1.1 mmol) was added to a solution of the enyne **S19** (200 mg, 0.72 mmol) in DMF (1.0 mL) and the resulting mixture was stirred at ambient temperature for 15 min. *tert*-Butyl bromoacetate (0.16 mL, 1.1 mmol) was introduced and the solution was stirred for 4 h. The reaction was quenched with sat. NH₄Cl solution (5 mL) and the aqueous phase was extracted with EtOAc (3 × 20 mL). The combined organic layers were washed with brine (2 × 10 mL), dried over MgSO₄ and evaporated. The crude product was purified by flash chromatography (silica, hexanes/EtOAc, 80:20) to give the title compound as a white solid (230.0 mg, 82 %).

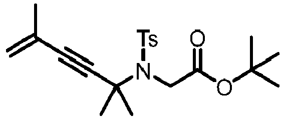

**N-(2,5-Dimethylhex-5-en-3-yn-2-yl)-N-(methoxymethyl)-4-methylbenzenesulfona-mide (S49).** A solution of enyne **S19** (300 mg, 1.08 mmol) in DMF (1 mL) was slowly added to a stirred suspension of NaH (39 mg, 1.6 mmol) in DMF (5 mL) at room temperature. The mixture was stirred for 30 min before MOMCI (0.18 mL, 2.4 mmol) was introduced. After stirring for 30 min at room temperature, sat. NH₄Cl solution (5 mL), water (5 mL) and *tert*-butyl methyl ether (30 mL) were added and the layers separated. The aqueous phase was extracted with *tert-*butyl methyl ether (2 × 30 mL) and the combined organic layers were washed with brine (3 × 10 mL) and dried over MgSO₄. The solvent was removed under reduced pressure and the crude product was purified by flash chromatography (silica, hexanes/EtOAc 20:1 - 10:1) to give the title compound as a colorless oil (290 mg, 83%).

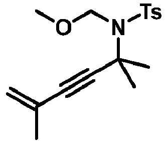

### Hydrogenative C-H Insertion Reactions

### Representative Procedure.

**Preparation of 4-(Cyclohex-1-en-1-yl)-2,2-dimethyltetrahydrofuran (7).** [Cp*RuCl]₄ (2.3 mg, 2 mol%) was added to a stirred solution of enyne **5** (19.0 mg, 0.11 mmol) in 1,2-dichloroethane (1.1 mL, 0.1 M) in a flame dried Schlenk tube under argon. H₂ was bubbled through the mixture for 2 min before the flask was immersed into a pre-heated oil bath (70 °C), keeping a static H₂ atmosphere (ambient pressure, balloon filled with H₂). After stirring for 3 h at 70 °C, the mixture was allowed to cool to room temperature, the solvent was removed under reduced pressure, and the crude product was purified by flash chromatography (silica, pentane/Et₂O 1:0 - 40:1) to yield the title compound as a colorless oil (17.4 mg, 89%).

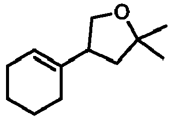

**4-(Cyclohex-1-en-1-yl)-2,2-dimethyltetrahydrofuran-3,3-[D₂] ([D₂]-7**) Prepared according to the Representative Procedure from enyne **5** using D₂ instead of H₂ gas (19.1 mg, 0.11 mmol); colorless oil (15.9 mg, 81%).

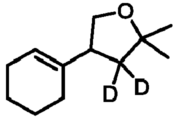

**4-(Cyclohex-1-en-1-yl)-2,2-dimethyltetrahydrofuran-4,5,5-[D₃] ([D₃]-7).** Prepared according to the Representative Procedure from enyne **[D₃]-5** (24.3 mg, 0.13 mmol); colorless oil (19.0 mg, 77%).

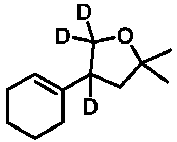

**Gram-Scale Reaction. Preparation of *tert*-Butyl 7-(prop-1-en-2-yl)-5-oxa-2-azaspiro[3.4]octane-2-carboxylate (20).** A flame dried two-necked round-bottom flask was charged with [Cp*RuCl]₄ (183 mg, 0.17 mmol). The flask was evacuated and refilled with H₂ (by means of attaching a balloon filled with hydrogen via a needle and septum). A solution of enyne **S25** (2.12 g, 8.43 mmol) in 1,2-dichloroethane (85 mL) was introduced before the flask was immersed into a pre-heated oil bath (70 °C), while keeping a static H₂ atmosphere (ambient pressure, balloon filled with H₂). After stirring for 3 h at 70 °C, the mixture was allowed to cool to room temperature, the flask was vented, and the solvent was removed under reduced pressure. The crude product was purified by flash chromatography (silica, hexanes/EtOAc 20:1 - 10:1) to yield the title product as a pale yellow oil (1.53 g, 72%).

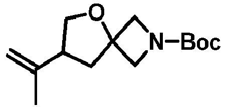

**(*E*)-(2-(1-Oxaspiro[4.5]decan-3-yl)vinyl)trimethylsilane (10).** According to the Representative Procedure from enyne **8** (26.9 mg, 0.11 mmol); colorless oil (22.3 mg, 92%).

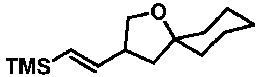

**4-(Cyclohex-1-en-1-yl)-5-methoxy-2,2-dimethyltetrahydrofuran** (11). According to the Representative Procedure from enyne **S23** (32.2 mg, 0.15 mmol); colorless oil (26.8 mg, 82%, mixture of diastereomers ~ 2:1).

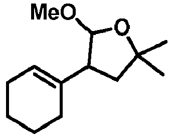

**3-(Prop-1-en-2-yl)-1-oxaspiro[4.4]nonane (12).** According to the Representative Procedure from enyne **S20** (32.2 mg, 0.20 mmol); colorless oil (22.9 mg, 70%).

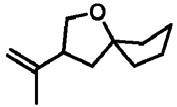

**7-(Cyclohex-1-en-1-yl)-5-oxaspiro[3.4]octane (13).** According to the Representative Procedure from enyne **S21** (18.6 mg, 0.10 mmol); colorless oil (9.7 mg, 52%, 75% NMR).

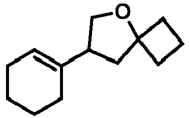

**3-(Prop-1-en-2-yl)-8-tosyl-1-oxa-8-azaspiro[4.5]decane (14).** According to the Representative Procedure from enyne **S26** (37.1 mg, 0.11 mmol); colorless oil (30.2 mg, 81%). Single crystals suitable for X-ray analysis were obtained by slow evaporation of a concentrated solution in CH₂Cl₂/pentane (1:1).

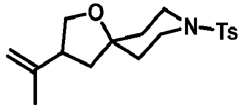

**3-(Prop-1-en-2-yl)-1,8-dioxaspiro[4.5]decane (15).** According to the Representative Procedure from enyne **38** (21.5 mg, 0.12 mmol); colorless oil (14.9 mg, 67%).

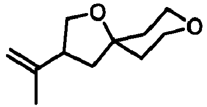

**8,8-Dimethyl-3-(prop-1-en-2-yl)-1,7,9-trioxaspiro[4.5]decane (16).** According to the Representative Procedure from enyne **S27** (22.7 mg, 0.11 mmol); colorless oil (18.0 mg, 79%).

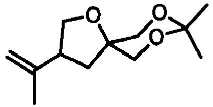

**(5*R*,6*S*,9*R*)-6-Isopropyl-9-methyl-3-(prop-1-en-2-yl)-1-oxaspiro[4.5]decane (17).** According to the Representative Procedure from enyne **S22** (21.3 mg, 0.09 mmol), colorless oil (7.8 mg, 36%; single isomer, isopropenyl stereochemistry unknown ).

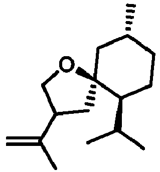

**Ethyl (E)-3-(1-oxaspiro[4.5]decan-3-yl)acrylate (18).** According to the Representative Procedure from enyne **S32** (28.2 mg, 0.12 mmol); colorless oil (18.2 mg, 64%).

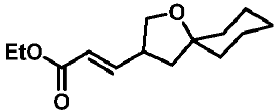

**(1-(1-Oxaspiro[4.5]decan-3-yl)vinyl)trimethylsilane (19).** According to the Representative Procedure from enyne **S33** (25.5 mg, 0.11 mmol); colorless oil (21.4 mg, 83%).

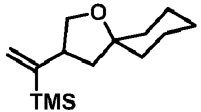

***tert*-Butyl 7-(prop-1-en-2-yl)-5-oxa-2-azaspiro[3.4]octane-2-carboxylate-6,6,7,8,8-[D₅] ([D_{5]}-20).** According to the Representative Procedure from enyne **[D₃]-S25** (33.6 mg, 0.13 mmol) but using D₂ instead of H₂ gas; colorless oil (23.5 mg, 69%).

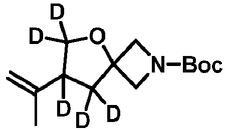

***tert*-Butyl 7-(3,3-diethoxyprop-1-en-2-yl)-5-oxa-2-azaspiro[3.4]octane-2-carboxylate (21).** Prepared analogously from enyne **S34** (30.7 mg, 0.090 mmol); colorless oil (27.3 mg, 88 %).

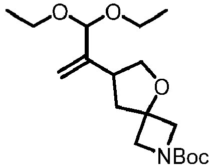

***tert*-Butyl 7-(4-((*tert*-butyldimethylsilyl)oxy)but-1-en-2-yl)-5-oxa-2-azaspiro[3.4]octane-2-carboxylate (22).** Prepared analogously from from enyne **S35** (36.0 mg, 0.091 mmol), colorless oil (26.0 mg,

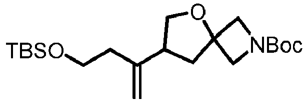

**Di-*tert*-butyl (6S*,7R*)-7-(prop-1-en-2-yl)-5-oxa-2-azaspiro[3.4]octane-2,6-dicarboxylate (23).** According to the Representative Procedure from enyne **S24** (44.2 mg, 0.13 mmol), colorless oil (36.1 mg containing 10% *trans*-hydrogenation product, 73%). An aliquot (26.4 mg) was purified by HPLC to yield an analytically pure sample (19.8 mg).

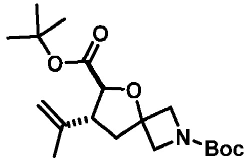

***tert*-Butyl 9-(prop-1-en-2-yl)-7-oxa-2-azadispiro[3.1.4⁶.1⁴]undecane-2-carboxylate (24).** According to the Representative Procedure from enyne **S28** (36.0 mg, 0.12 mmol); colorless oil (27.7 mg, 76%).

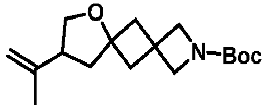

***tert*-Butyl (*E*)-7-(3-((*tert*-butyldimethylsilyl)oxy)prop-1-en-1-yl)-5-oxa-2-azaspiro[3.4] octane-2-carboxylate (25).** Prepared analogously from enyne **S29** (39.7 mg, 0.10 mmol); colorless oil (18.0 mg 45%).

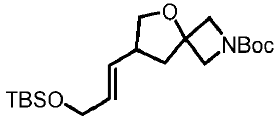

***tert*-Butyl 7-(3,3,3-trifluoroprop-1-en-2-yl)-5-oxa-2-azaspiro[3.4]octane-2-carboxylate (26).** Prepared analogously from enyne **S36** (32.7 mg, 0.11 mmol); pale yellow oil (11.0 mg, 36 %).

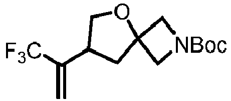

**1-Cyclohexyl-6-(prop-1-en-2-yl)-2,8-dioxabicyclo[3.2.1]octane (31).** Prepared analogously from enyne **30** (26.4 mg, 0.11 mmol); colorless oil (21.8 mg, 82 %, mixture of diastereoisomers ~ 2.5:1).

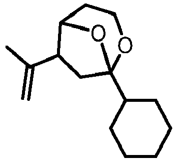

**(3S*,5R*)-3-(Prop-1-en-2-yl)-1,6-dioxaspiro[4.4]nonane (33).** According to the Representative Procedure B from enyne **32** (34.4 mg, 0.21 mmol); colorless oil (26.2 mg, 75%).

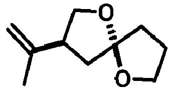

**(2R*,4R*)-2-Cyclohexyl-2-methoxy-4-(prop-1-en-2-yl)tetrahydrofuran (34).** Prepared analogously from enyne **S40** (30.7 mg, 0.14 mmol); colorless and *very acid-sensitive* oil (21.2mg, 68 %).

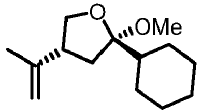

**(1R*,4R*,5R*)-1-Cyclohexyl-5-(prop-1-en-2-yl)-2,7-dioxabicyclo[2.2.1]heptane (35).** Prepared analogously from enyne **S41** (22.0 mg, 0.10 mmol); colorless, *acid sensitive* oil (11.0 mg, 49 %).

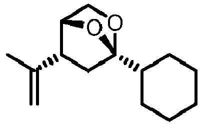

**(4*S**,5*S**)-4-(Cyclohex-1-en-1-yl)-2,2-dimethyl-1,6-dioxaspiro[4.5]decane (36a) and (4*S**,5*R**)-4-(cyclohex-1-en-1-yl)-2,2-dimethyl-1,6-dioxaspiro[4.5]decane (36b**) Prepared analogously from enyne **S43** (30.7 mg, 0.090 mmol); flash chromatography (silica, hexane:*tert*-butyl methyl ether, 98:2) allowed the diastereoisomers to be isolated in analytically pure form.
**36a:** Colorless oil (8.7 mg, 31 %); **36b:** Colorless oil (7.2 mg, 27 %).

**(3S*,5R*)-2,2-Dimethyl-3-(prop-1-en-2-yl)-1,6-dioxaspiro[4.4]nonane (37).** According to the Representative Procedure from enyne **S42** (39.1 mg, 0.20 mmol); colorless oil (25.6 mg, 65%).

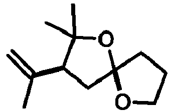

**(1S*,5S*,7S*)-7-(Prop-1-en-2-yl)-5-((trimethylsil)oxy)-2-oxabicyclo[3.2.1]octane (40).** Prepared analogously from enyne **39** (23.8 mg, 0.1 mmol); colorless oil (16.9 mg, 70 %).

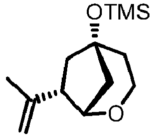

**3-(Prop-1-en-2-yl)-1,7-dioxaspiro[4.4]nonane (41).** Prepared analogously from enyne **S31** (29.2, 0.18 mmol); colorless oil (19.9 mg, 68 %, mixture of diastereoisomers ~ 1.2:1).

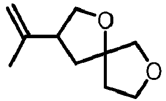

***tert*-Butyl 3-(prop-1-en-2-yl)-1-oxa-7-azaspiro[4.4]nonane-7-carboxylate (42)**. Prepared analogously from enyne **S30** (26:0 mg, 0.098 mmol); colorless oil (20.0 mg, 77% (contains 5% of an unknown impurity), mixture of diastereoisomers 1:1). Analysis is further complicated by the fact that the signals in the ¹³C NMR spectra of each diastereoisomer are split (ca. 1:1) due to the presence of rotamers of the Boc group. 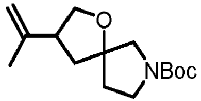 Repeated flash chromatography furnished a pure sample of one diastereoisomers, whereas the second isomer is contaiminated by the mentioned impurity of unknown composition, see copies of Spectra provided below

**(1*S**,5*S**,7*S**)-7-(Prop-1-en-2-yl)-2-tosyl-5-((trimethylsilyl)oxy)-2-azabicyclo[3.2.1]octane (43).** Prepared analogously from enyne **S44** (38.8 mg, 0.099 mmol) but using 4 mol% of [Cp*RuCl]₄ (30.0 mg, 77

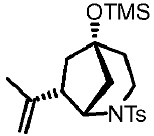

**6-(Prop-1-en-2-yl)-4-((trimethylsilyl)oxy)-2-oxabicyclo[2.2.1]heptane (44).** Prepared analogously from enyne **S45** (27.7 mg, 0.12 mmol); colorless oil (14.0 mg, 51 %).

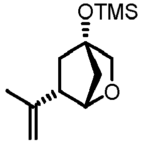

***tert*-Butyl 4-hydroxy-6-(prop-1-en-2-yl)-2-azabicyclo[2.2.1]heptane-2-carboxylate (45).** [Cp*RuCl]₄ (5.4 mg, 4 mol%) was added to a stirred solution of enyne **S46** (40.8 mg, 0.13 mmol) in 1,2-dichloroethane (1.6 mL, 0.1 M) in a flame-dried Schlenk tube under argon. H₂ was bubbled through the mixture for 2 min before the flask was immersed into a pre-heated oil bath at 70 °C keeping a static H₂ atmosphere (ambient pressure, balloon filled with H₂). After stirring for 3 h at 70 °C, the mixture was cooled to room temperature and then filtered hrough a plug of Florisil, eluting with Et₂O (10 mL). The filtrate was evaporated and the residue redissolved in THF (2.0 mL),
TBAF·3H₂O (79.5 mg, 0.25 mmol) was added to this solution of the crude material and the mixture was stirred at or 2h before it was poured in water. The aqueous phase was extracted with Et₂O (3 × 5 mL), the combined organic layers were dried over MgSO₄ and evaporated, and the residue was purified by flash chromatography (silica, Hex/EtOAc 6:4) to yield the title product as a colorless oil (25.9 mg, 81%).

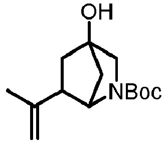

**2,2-Dimethyl-4-(prop-1-en-2-yl)-1-tosylpyrrolidine (47).** Prepared analogously from enyne 46 (56.9 mg, 0.195 mmol); white solid (52.1 mg, 90 %).

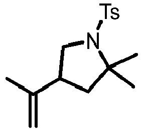

***tert*-Butyl 2,2-dimethyl-4-(prop-1-en-2-yl)pyrrolidine-1-carboxylate (48).** According to the Representative Procedure from enyne **S47** (30.8 mg, 0.13 mmol); colorless oil (17.2 mg, 55%).

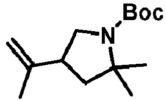

***tert*-Butyl (2*R**,3*R**)5,5-dimethyl-3-(prop-1-en-2-yl)-1-tosylpyrrolidine-2-carboxylate (49).** Prepared analogously from enyne **S48** (38.5 mg, 0.10 mmol); colorless oil (26.0 mg, 67 %).

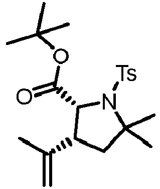

**(4*R**,5*S**)5-Methoxy-2,2-dimethyl-4-(prop-1-en-2-yl)-1-tosylpyrrolidine (50)** Prepared analogously from enyne **S49** (33.5 mg, 0.10 mmol); colorless oil (20.1 mg, 60 %).

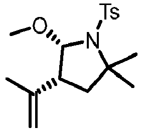

**5,5,5-Trimethoxy-2-methylpent-1-en-3-yne (58).** *n*BuLi (1.79 mL, 2.86 mmol, 1.6 M in hexanes) was slowly added to a solution of 2-methyl-1-buten-3-yne (0.27 mL, 2.86 mmol) in THF (120 mL) at -78°C, and the resulting mixture was stirred for 30 min. In parallel, BF₃·Et₂O (0.74 mL, 2.86 mmol) was added dropwise to a solution of tetramethyl orthocarbonate (0.40 mL, 3.01 mmol) in Et₂O (20 mL) at -78°C. After the addition, the temperature was raised to 0°C for 1h and the mixture finally cooled again to -78°C. The solution of the organolithium reagent was then added via cannula to the solution of the oxonium salt. After the addition was complete, the mixture was stirred for 1h at -78°C and for another 1h at 25°C. Finally the mixture was cooled to -20°C before it was poured into a solution of Na₂CO₃ (70 mL) at 0°C. The aqueous phase was extracted with CH₂Cl₂ (3 × 50 mL), the combined organic layers were dried over Na₂SO₄, filtrated and concentrated to give the desired product as a colorless oil, which was used without further purification (377 mg, 77%).

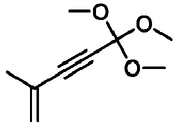

**5,5,5-Triethoxy-2-methylpent-1-en-3-yne (61).** Prepared analogously from tetraethyl orthocarbonate as a colorless oil;

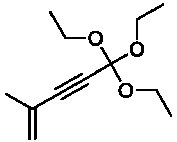

**2,2-Dimethoxy-4-(prop-1-en-2-yl)tetrahydrofuran (59).** [(η⁵-Cp*)RuCl]₄ (8.9 mg, 2 mol%) was added to a stirred solution of 5,5,5-trimethoxy-2-methylpent-1-en-3-yne (70.0 mg, 0.41 mmol) in 1,2-dichloroethane (4.1 mL) in a flame-dried Schlenk flask under argon. H₂ was bubbled through the mixture for 2 min before the flask was immersed into a pre-heated oil bath at 70°C, keeping a static H₂ atmosphere (ambient pressure, H₂ filled balloon). After stirring for 3h at 70°C, the mixture was allowed to cool to room temperature, the solvent was removed under reduced pressure and the crude product was purified by flash chromatography (silica, hexane/ EtOAc) to give the desired product as a colorless oil (53.8 mg, 76%).

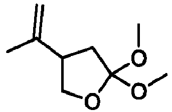

**Methyl-4-(prop-1-en-2-yl)dihydrofuran-2(3H)-one (60).** *p*-Toluenesulfonic acid monohydrate (3.1 mg, 0.016 mmol) was added to a stirred solution of 2,2-di-methoxy-4-(prop-1-en-2-yl)tetrahydrofuran (28.0 mg, 0.16 mmol) in acetone (1.0 mL) in a flame dried Schlenk tube under argon. The mixture was vigorously stirred at 25°C for 4 h. The mixture was concentrated and the residue purified by flash chromatography (silica, CH₂Cl₂) to give the desired product as a colorless oil (16.9 mg, 82%).

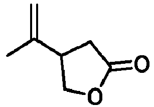

**5-Methyl-4-(prop-1-en-2-yl)dihydrofuran-2(3*H*)-one (62).** [(η⁵-Cp*)RuCl]₄ (22.0 mg, 2 mol%) was added to a stirred solution of 5,5,5-triethoxy-2-methylpent-1-en-3-yne (213 mg, 1.00 mmol) in 1,2-dichloroethane (10 mL) in a flame dried Schlenk tube under argon. H₂ was bubbled through the mixture for 2 min before the flask was immersed into a pre-heated oil bath at 70°C keeping a static H₂ atmosphere (ambient pressure, H₂ filled balloon). After stirring for 5 h at 70°C, the mixture was allowed to cool to room temperature, the solvent was removed under reduced pressure.

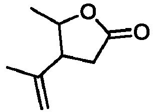

The residue was purified by flash chromatography (silica, hexane/EtOAc) to give the desired product as a colorless oil (87.3 mg, 62%).

### Conclusion

As discussed above, the inventive hydrogenative C-H insertion process is a conceptually novel mode of H₂-transfer to an organic substrate, which the inventor's group was able to discover after a century of intense research devoted to catalytic hydrogenation in innumerous academic as well as industrial laboratories. The present invention shows that 1,3-enynes bearing a propargylic substituent are amenable to this process preferably using [Cp*RuCl]₄ as the catalyst. The ensuing reaction is highly enabling in preparative terms; most notably, it provides ready access to spirocyclic as well as bridged ring systems of immediate relevance as building blocks for medicinal chemistry and chemical biology. The process scales well and lends itself to the preparation of deuterated isotopologues. This novel hydrogenative C-H insertion process hence provides a notable addendum to the growing list of reactions exploiting gem-hydrogenation as a means to generate reactive intermediates and augurs well for further explorations of this field of research.

## Claims

1. Process for C-H insertion by gem-hydrogenation of an internal alkyne wherein a compound of Formula (I) is hydrogenated in the presence of a Ru-catalyst in an organic solvent, whereby a compound of Formula (II) is obtained: 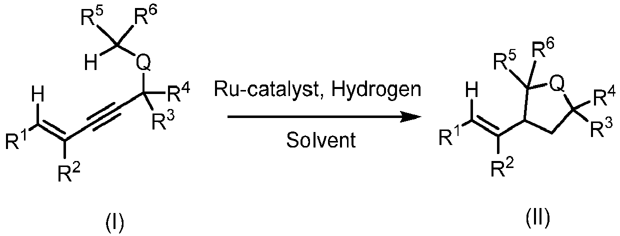 wherein in Formulae (I) and (II):
R¹ and R² each independently represent
∘ hydrogen, optionally with the proviso that at least one of R¹ and R² is not hydrogen;
∘ Si(C₁ to C₁₂ alkyl)₃, C(=O)-O-(C₁ to C₁₂ alkyl), or
∘ (C₁ to C₁₂)alkyl or hetero(C₁ to C₁₂)alkyl, wherein R¹ and R² may form a ring system with each other via the alkyl or the heteroalkyl,
R³ and R⁴ each independently represent
∘ OSi(C₁ to C₁₂ alkyl)₃, C(=O)-O-(C₁ to C₁₂ alkyl); or
∘ (C₁ to C₁₂)alkyl or hetero(C₁ to C₁₂)alkyl, wherein R³ and R⁴ may form a ring system with each other via the alkyl or the heteroalkyl;
R⁵ and R⁶ each independently represent
∘ hydrogen, deuterium,
∘ (C₁ to C₁₂)alkyl, C(=O)-(C₁ to C₁₂ alkyl), C(=O)-O-(C₁ to C₁₂ alkyl),
∘ NR^{N1}R^{N2}, wherein R^{N1} represents a protective group selected from an arylsulfonyl group, a (C₁ to C₁₂)alkylsulfonyl group, a (C₁ to C₁₂)alkoxy carbonyl group or -C(=O)-(C₁ to C₁₂ alkyl), and R^{N2} represents a C₁ to C₁₂ alkyl group, or wherein R^{N1} and R^{N2} each represent a C₁ to C₁₂ alkyl group, or
∘ (C₁ to C₁₂) alkyl or hetero(C₁ to C₁₂)alkyl, wherein R⁵ and R⁶ may form a ring system with each other via the alkyl or the heteroalkyl,
wherein R² and R³ may form a ring system with each other if R₁ and R₂ do not form a ring with each other, and/or R⁴ and R⁵ may form a ring system with each other if R₃ and R₄ do not form a ring with each other;
wherein Q represents CH₂, CH(C₁ to C₁₂ alkyl), C(C₁ to C₁₂ alkyl)₂, O or NR^{N} wherein
R^{N} represents a protective group selected from an arylsulfonyl group, a (C₁ to C₁₂)alkylsulfonyl group, a (C₁ to C₁₂)alkoxy carbonyl group or -C(=O)-(C₁ to C₁₂ alkyl), with the proviso that, if Q represents CH₂, CH(C₁ to C₁₂ alkyl), or C(C₁ to C₁₂ alkyl)₂,
R⁴ and R⁵ each independently represent (C₁ to C₁₂)alkyl or hetero(C₁ to C₁₂)alkyl and form a ring system with each other with O or NR^{N} in the ring wherein R^{N} represents a protective group selected from an arylsulfonyl group, a (C₁ to C₁₂)alkylsulfonyl group, a (C₁ to C₁₂)alkoxy carbonyl group or -C(=O)-(C₁ to C₁₂ alkyl),
R³ represents C₁ to C₁₂ alkyl, hetero(C₁ to C₁₂)alkyl or OSi(C₁ to C₁₂ alkyl)₃,
R⁶ represents hydrogen, deuterium, C₁ to C₁₂ alkyl or hetero(C₁ to C₁₂)alkyl,, and
R¹ and R² each independently represent:
∘ hydrogen, optionally with the proviso that at least one of R¹ and R² is not hydrogen;
∘ Si(C₁ to C₁₂ alkyl)₃, C(=O)-O-(C₁ to C₁₂ alkyl), or
∘ (C₁ to C₁₂)alkyl or hetero(C₁ to C₁₂)alkyl,
wherein the Ru catalyst is represented by the Formula (III) 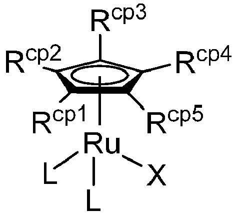
wherein R^{cp1} to R^{cp5} each independently represents C₁-C₅ alkyl which may be branched or linear, C₃-C₅ cycloalkyl, OR^{H} or NR^{H}₂, C(=O)-O-(C₁ to C₁₂ alkyl),
C(=O)N(C₁ to C₁₂ alkyl)₂, X represents Cl, Br, I, OTf, BF₄, PF₆, O(C₁ to C₁₂ alkyl) and
L is a ligand, or a di-, tri- or tetramer thereof such as [Cp*RuCl]₄.

2. Process for C-H insertion by gem-hydrogenation of an internal alkyne according to claim 1, wherein the Ru-catalyst is selected from from COD, NBD, (C₁ to C₁₂ alkyl)CN, or (C₁ to C₁₂ alkyl)₂O, tetrahydrofuran, tetrahydropyran, 1,4-dioxane, substituted pyridines, η-arenes, H₂ and further common readily dissociating dative ligands known in the art, preferably [Cp*RuCl]₄ and wherein R¹ to R⁶ and Q are as defined in claim 1.

3. Process for C-H insertion by gem-hydrogenation of an internal alkyne according to claim 1 or 2, wherein the compound of Formula (I) is hydrogenated in the presence of a Ru-catalyst in an organic solvent, wherein, in Formula (I):
R³ and R⁴ each independently represent (C₁ to C₁₂)alkyl or (hetero)(C₁ to C₁₂)alkyl and
R³ and R⁴ preferably form a ring system with each other via the alkyl or the heteroalkyl,
R¹ and R² each independently represent
∘ hydrogen, optionally with the proviso that at least one of R¹ and R² is not hydrogen;
∘ Si(C₁ to C₁₂ alkyl)₃, C(=O)-O-(C₁ to C₁₂ alkyl), or
∘ (C₁ to C₁₂)alkyl or hetero(C₁ to C₁₂)alkyl, and wherein R¹ and R² may form a ring system with each other via the alkyl or the heteroalkyl,
R⁵ and R⁶ each independently represent
∘ hydrogen, deuterium,
∘ O(C₁ to C₁₂ alkyl), C(=O)-(C₁ to C₁₂ alkyl), C(=O)-O-(C₁ to C₁₂ alkyl),
∘ NR^{N1}R^{N2}, wherein R^{N1} represents a protective group selected from an arylsulfonyl group, a (C₁ to C₁₂)alkylsulfonyl group, a (C₁ to C₁₂)alkoxy carbonyl group or -C(=O)-(C₁ to C₁₂ alkyl), and R^{N2} represents a C₁ to C₁₂ alkyl group, or wherein R^{N1} and R^{N2} each represent a C₁ to C₁₂ alkyl group, or
∘ (C₁ to C₁₂)alkyl or hetero(C₁ to C₁₂)alkyl, wherein R⁵ and R⁶ may form a ring system with each other via the alkyl or the heteroalkyl, and
wherein Q represents O or NR^{N} wherein R^{N} represents a protective group selected from an arylsulfonyl group, a (C₁ to C₁₂)alkylsulfonyl group, a (C₁ to C₁₂)alkoxy carbonyl group or -C(=O)-(C₁ to C₁₂ alkyl).

4. Process for C-H insertion by gem-hydrogenation of an internal alkyne according to claim 1 or 2, wherein the compound of Formula (I) is hydrogenated in the presence of a Ru-catalyst in an organic solvent, wherein, in Formula (I):
R³ and R⁴ each independently represent a (C₁ to C₁₂)alkyl or hetero(C₁ to C₁₂)alkyl and R³ and R⁴ preferably form a ring system with each other via the alkyl or the heteroalkyl,
R⁵ and R⁶ each independently represent hydrogen, deuterium, (C₁ to C₁₂)alkyl or
hetero(C₁ to C₁₂)alkyl,
R¹ and R² each independently represent
∘ hydrogen, optionally with the proviso that at least one of R¹ and R² is not hydrogen;
∘ Si(C₁ to C₁₂ alkyl)₃, C(=O)-O-(C₁ to C₁₂ alkyl), or
∘ (C₁ to C₁₂)alkyl or hetero(C₁ to C₁₂)alkyl, wherein R¹ and R² may form a ring system with each other via the alkyl or the heteroalkyl, and
wherein Q represents O.

5. Process for C-H insertion by gem-hydrogenation of an internal alkyne according to claim 1 or 2, wherein the compound of Formula (I) ) is hydrogenated in the presence of a Ru-catalyst in an organic solvent, wherein, in Formula (I),
R³ and R⁴ each independently represent a (C₁ to C₁₂)alkyl or hetero(C₁ to C₁₂)alkyl and R³ and R⁴ preferably form a ring system with each other via the alkyl or the heteroalkyl,
R¹ and R² each independently represent
∘ hydrogen, optionally with the proviso that at least one of R¹ and R² is not hydrogen;
∘ Si(C₁ to C₁₂ alkyl)₃, C(=O)-O-(C₁ to C₁₂ alkyl), or
∘ (C₁ to C₁₂)alkyl or hetero(C₁ to C₁₂)alkyl, wherein
R¹ and R² may form a ring system with each other via the alkyl or the heteroalkyl,
R⁵ and R⁶ each independently represent
∘ hydrogen, deuterium,
∘ O(C₁ to C₁₂ alkyl), C(=O)-(C₁ to C₁₂ alkyl), C(=O)-O-(C₁ to C₁₂ alkyl),
∘ NR^{N1}R^{N2}, wherein R^{N1} represents a protective group selected from a arylsulfonyl group, a (C₁ to C₁₂)alkylsulfonyl group, a alkoxy carbonyl group or -C(=O)-(C₁ to C₁₂ alkyl), and R^{N2} represents a C₁ to C₁₂ alkyl group, or wherein R^{N1} and R^{N2} each represent a C₁ to C₁₂ alkyl group, or
∘ (C₁ to C₁₂)alkyl or hetero(C₁ to C₁₂)alkyl, wherein R⁵ and R⁶ may form a ring system with each other via the alkyl or the heteroalkyl, and
wherein Q represents NR^{N} wherein R^{N} represents a protective group selected from a arylsulfonyl group, a (C₁ to C₁₂)alkylsulfonyl group, a alkoxy carbonyl group or - C(=O)(C₁ to C₁₂ alkyl), I.

6. Process for C-H insertion by gem-hydrogenation of an internal alkyne according to claim 1 or 2, wherein the compound of Formula (I) is hydrogenated in the presence of a Ru-catalyst in an organic solvent, wherein, in Formula (I):
R¹ and R² each independently represent
∘ hydrogen, optionally with the proviso that at least one of R¹ and R² is not hydrogen;
∘ Si(C₁ to C₁₂ alkyl)₃, C(=O)-O-(C₁ to C₁₂ alkyl), or
∘ (C₁ to C₁₂)alkyl or hetero(C₁ to C₁₂)alkyl,
wherein R¹ and R² may form a ring system with each other via the alkyl or the heteroalkyl,
R³ and R⁴ each independently represent O(C₁ to C₁₂)alkyl,
R⁵ represents hydrogen and R⁶ represents hydrogen or (C₁ to C₁₂)alkyl, and
wherein Q represents O.

7. Process for C-H insertion by gem-hydrogenation of an internal alkyne according to claim 1 or 2, wherein the compound of Formula (I) is hydrogenated in the presence of a Ru-catalyst in an organic solvent, wherein, in Formula (I):
R⁴ and R⁵ each independently represent (C₁ to C₁₂)alkyl or hetero(C₁ to C₁₂)alkyl and preferably form a ring system with each other with O or NR^{N} in the ring wherein R^{N} represents a protective group selected from an arylsulfonyl group, a (C₁ to C₁₂)alkylsulfonyl group, a (C₁ to C₁₂)alkoxy carbonyl group or -C(=O)-(C₁ to C₁₂ alkyl),
R³ represents (C₁ to C₁₂)alkyl, hetero(C₁ to C₁₂)alkyl or OSi(C₁ to C₁₂ alkyl)₃,
R⁶ represents hydrogen, deuterium, (C₁ to C₁₂)alkyl or hetero(C₁ to C₁₂)alkyl,, and
R¹ and R² each independently represent:
∘ hydrogen, optionally with the proviso that at least one of R¹ and R² is not hydrogen;
∘ Si(C₁ to C₁₂ alkyl)₃, C(=O)-O-(C₁ to C₁₂ alkyl), or (C₁ to C₁₂)alkyl or hetero(C₁ to C₁₂)alkyl.. and
wherein Q represents CH₂, CH(C₁ to C₁₂ alkyl), or C(C₁ to C₁₂ alkyl)₂.

8. Process for C-H insertion by gem-hydrogenation of an internal alkyne according to claim 1 or 2, wherein the compound of Formula (I)

9. Process for C-H insertion by gem-hydrogenation of an internal alkyne according to any one of the preceding claims, wherein the compound of Formula (I) is hydrogenated in the presence of a Ru-catalyst in an organic solvent preferably selected from aprotic apolar organic solvents.

10. Process for C-H insertion by gem-hydrogenation of an internal alkyne according to any one of the preceding claims, wherein the compound of Formula (I) is hydrogenated in the presence of a Ru-catalyst in an organic solvent under a H₂ partial pressure of 0,1 to 100 atm, preferably 0,5 to 5 bar and more preferred 0,8 atm to 1,5 atm.

11. Process for C-H insertion by gem-hydrogenation of an internal alkyne according to any one of the preceding claims, wherein the compound of Formula (I) is hydrogenated in the presence of a Ru-catalyst in an organic solvent in a temperature range of 0°C to 150°C, preferably in a temperature range of 60°C to 80°C.

12. Process for C-H insertion by gem-hydrogenation of an internal alkyne according to any one of the preceding claims, wherein the compound of Formula (I) is hydrogenated in an organic solvent in the presence of a Ru-catalyst in a molar amount of 1 to 10 mol%, referred to the molar amount of the compound of Formula (I).

13. Process for C-H insertion by gem-hydrogenation of an internal alkyne according to any one of the preceding claims, wherein the compound of Formula (I) is hydrogenated in the presence of a Ru-catalyst in an organic solvent over a reaction time of 30 min to 600 min, preferably 120 min to 240 min.
